# EUROPEAN PATENT APPLICATION

(11) **EP 4 703 725 A1**
(43) Date of publication of application: **04.03.2026**
(21) Application number: 24197432.8
(22) Date of filing: 29.08.2024
(51) Int. Cl.: G01N 33/50, G01N 33/574, G01N 33/68, A61K 39/395, C12N 5/10

(54) **MODULATORS OF THE MITOTIC CELL CYCLE IN CANCER CELLS AND USES THEREOF FOR THE TREATMENT OF CANCER**

(71) Applicant: Chemotherapeutisches Forschungsinstitut Georg-Speyer-Haus, 60596 Frankfurt am Main (DE); Johann Wolfgang Goethe-Universität Frankfurt, 60323 Frankfurt am Main (DE)
(72) Inventor: Greten, Florian, 61352 Bad Homburg (DE); CETECI, Faith, 60598 Frankfurt am Main (DE)
(74) Representative: Krauss, Jan

(57) **Abstract**

The present invention relates to a method for identifying a compound that modulates the mitotic cell cycle in cancer cells by modulating at least one of the expression, the amount, the stability and/or the biological activity of the protein CITED2 and/or CITED2 containing protein complexes in a mammalian cell. Further provided is a method for determining the level of CITED2 and/or CDK11b^{p58} and/or splicing factor 3B subunit 1 (SF3B1), and/or 14-3-3γ in a sample obtained from a subject, comprising detecting of binding of CITED2 in the sample to at least one molecule selected from the group consisting of CDK11b^{p58}, splicing factor 3B subunit 1 (SF3B1), and 14-3-3γ, wherein a decrease of the binding, when compared to a control indicates an improper centrosome segregation and subsequent an increase of the formation of chaotic monopolar mitotic spindles in said sample. Further provided are pharmaceutical compositions comprising the compound as identified for use in medicine, in particular for use in the prevention or treatment of a disease or condition caused by cancer.

## Description

The present invention relates to a method for identifying a compound that modulates the mitotic cell cycle in cancer cells by modulating at least one of the expression, the amount, the stability and/or the biological activity of the protein CITED2 and/or CITED2 containing protein complexes in a mammalian cell. Further provided is a method for determining the level of CITED2 and/or CDK11b^{p58} and/or splicing factor 3B subunit 1 (SF3B1), and/or 14-3-3γ in a sample obtained from a subject, comprising detecting of binding of CITED2 in the sample to at least one molecule selected from the group consisting of CDK11b^{p58}, splicing factor 3B subunit 1 (SF3B1), and 14-3-3γ, wherein a decrease of the binding, when compared to a control indicates an improper centrosome segregation and subsequent an increase of the formation of chaotic monopolar mitotic spindles in said sample. Further provided are pharmaceutical compositions comprising the compound as identified for use in medicine, in particular for use in the prevention or treatment of a disease or condition caused by cancer.

### Background of the invention

Precise control of intestinal stem cell (ISC) proliferation and survival is essential for maintenance of gut tissue integrity and function. Under homeostatic conditions, actively dividing Lgr5⁺ crypt base columnar cells (CBCs) that are interspersed among the Paneth cells at the bottom part of the crypt constantly fuel renewal of the epithelium in the intestinal tract (1). Lgr5⁺ stem cells can divide to form TA cells, which undergo several rounds of cell division before differentiating and losing proliferative capacity (2). It was shown that Lgr5⁺ cells have a significantly longer cell-cycle than TA cells, indicating a differential regulation of the cell-cycle among the crypt cell populations (3).

Another major difference between intestinal Lgr5⁺ and TA cells lies in their response to stress signals. Whereas Lgr5⁻ intestinal progenitor cells preferentially survive in the context of DNA damage, Lgr5⁺ CBC cells equipped with high Wnt signaling activity were found to be exquisitely sensitive to DNA damage responses (4).

The maintenance of genome integrity, one of the hallmarks of sternness in mammalian tissues, is key to preserving function of stem cells and reducing the risks of cancer development, cell cycle arrest and abnormal replication (5). Despite the identity of rapidly cycling and infrequently dividing (quiescence) stem cell populations in the intestine, factors modulating their faithful mitosis and protection from accumulation of DNA damage are largely unknown.

CITED2 is a member of the CITED2 (CBP/p300-interacting transactivator with ED tail-rich C-terminal domain) family of nuclear proteins. Although it does not bind DNA itself, CITED2 acts as transcriptional modulator through the recruitment of the histone acetyltransferases CBP/p300 (6, 7, 8). CITED2 controls proliferation of mouse embryonic fibroblasts (MEFs) via polycomb group genes *Bmi-1* and *Mel18* and the tumor suppressor *Ink4a*/ *Arf* (9). CITED2 has been shown to sustain both self-renewal and proliferation of mouse embryonic stem cell (ESC) cultured in the absence of LIF and its acute loss impaired ESC proliferation and survival (10, 11, 12). Loss of CITED2 in mice results in embryonic lethality due to multiple developmental defects (7, 13, 14). In the adult mouse acute *Cited2* deletion causes loss of HSCs leading to multilineage bone marrow failure and increased lethality (15, 16) while CITED2 expression has been correlated with maintenance of human and rat adult tendon stem and progenitor cells (17, 18).

Collectively, these findings indicate an association between CITED2 loss and impaired adult tissue stem cell function. However, the functional role of CITED2 in intestinal stem and progenitor cells has not been determined.

WO 2017/173206A1 describes a method for modulating the levels of a biomarker in a subject having a cancer, the method comprising administering to the subject an effective amount of an aminopurine compound, wherein the biomarker can be CITED2. Nevertheless, the publication desires a decrease in mRNA or protein expression levels of CITED2.

WO 2010/030365A2 describes CITED2 as a gene that is expressed at lower levels in malignant thyroid cancer tissues than in benign thyroid lesions.

WO 2017/161188A1 describes CITED2 as upregulated in the context of anti-pd-1 therapy resistant metastatic melanomas.

Chou YT, et al. (in: CITED2 functions as a molecular switch of cytokine-induced proliferation and quiescence. Cell Death Differ. 2012 Dec; 19(12):2015-28. doi: 10.1038/cdd.2012.91. Epub 2012 Jul 20. PMID: 22814619; PMCID: PMC3504715) disclose that ectopic CITED2 expression enhanced tumor growth in nude mice; furthermore, CITED2 knockdown caused tumor shrinkage and increased overall host mouse survival rates. Expression of CITED2/MYC/E2F3/p21(CIP1) signaling molecules was associated with poor prognosis of lung cancer patients. Thus, CITED2 functions as a molecular switch of TGF-α and TGF-β-induced growth control, and MYC-CITED2 signaling axis could provide a new index for predicting clinical outcome.

Shin SH, et al. (in: Aberrant expression of CITED2 promotes prostate cancer metastasis by activating the nucleolin-AKT pathway. Nat Commun. 2018 Oct 5;9(1):4113. doi: 10.1038/s41467-018-06606-2. PMID: 30291252; PMCID: PMC6173745) disclose that CITED2 is highly expressed in metastatic prostate cancer, and its expression is correlated with poor survival. They suggest that CITED2 could be a potential target to prevent prostate cancer metastasis. Since the complete inhibition of CITED2 has been reported to induce acute bone marrow failure, the anti-CITED2 strategy should be carefully optimized before clinical application.

Despite all above progress in research of the association of CITED2 in cancer, potential CITED2 containing complexes, interaction partners of CITED2 and its functional role in the mitotic cell cycle, particularly in cancer cells has not be determined.

Consequently, the object of the present invention is to provide diagnostic tools, and to develop improved pharmaceutical compositions for treating and controlling the progression of cancer cells. Other objects and advantages of the present invention will become apparent to the person skilled in the art when studying the following more detailed description of the present invention, including the Figures and examples.

In a first aspect of the present invention, the problem of the present invention is solved by providing A method for identifying a compound that modulates the mitotic cell cycle in cancer cells by modulating at least one of the expression, the amount, the stability and/or the biological activity of the protein CITED2 and/or CITED2 containing protein complexes in a mammalian cell, comprising the steps of:
a) contacting at least one of CITED2 or a functional fragment thereof and/or a cell expressing CITED2 or the functional fragment thereof with at least one compound that potentially modulates at least one of the expression, the amount, the stability and/or the biological activity of the protein CITED2 in the mammalian cell, b) identifying a modulation of at least one of the expression, the stability and/or the biological activity of the protein CITED2 in the presence of the at least one compound, when compared to the absence of said at least one compound, and c) identifying a compound of step b) as modulating, in particular specifically modulating, the mitotic cell cycle in the cancer cells based on said modulation as identified in step b).

In view of the results as obtained in the experiments as performed in the context of the present invention, the inventors found that CITED2 exerts important functions in the mitotic machinery and prevents mitotic defects in intestinal epithelia. Furthermore, CITED2 expression is restricted to intestinal crypt cells and reminiscent of hematopoietic adult stem cells, loss of CITED2 in intestinal epithelium resulted in cell death in actively cycling Lgr5⁺ ISCs indicating a conserved function of CITED2 for promoting niche-specific essential survival signals (15).

In the context of the present invention, the term "CITED2" shall be understood as indicating/representing the mammalian, in particular human or ortholog/homolog of the human Cbp/p300-interacting transactivator 2 (CITED2) gene and/or protein and/or mRNA, in particular of the mouse. Also, the term shall comprise the complete CITED2 polypeptide or fragment(s) of the CITED2 polypeptide in different preparations, such as in the cellular context or purified from the cell. The human amino acid sequence of CITED2 (aliases: MSG-related protein 1 (MRG-1), and P35srj) can be, e.g. found at UniProt Q99967.

In the context of the present invention, the term "CITED2 containing complexes" shall be understood as a stable assembly of two or more macromolecules, i.e. proteins, nucleic acids, carbohydrates and/or lipids, in which at least one component is the protein CITED2, and wherein preferably the components together have a biological function.

In the context of the present invention, the term "functional fragment of CITED2" shall relate to a part of the protein or the amino acid sequence of the above protein that is truncated at the N- and/or C-terminus of the amino acid sequence, but still performs and/or supports its function as required/desired in the context of the present invention, namely - when expressed, translated and/or administered - may be oxidized, and binds as disclosed herein.

Preferred is the method according to the present invention, wherein said modulation is selected from a decrease or an increase of the expression, the amount, the stability and/or the biological activity of the protein CITED2 in a mammalian cell, preferably an inhibition of the expression, the amount, the stability and/or the biological activity of the protein CITED2, wherein the biological activity is preferably selected from the interaction of CITED2 with at least one molecule selected from the group consisting of CDK11b^{p58}, splicing factor 3B subunit 1 (SF3B1) and 14-3-3γ.

The term "contacting" in the present invention means any interaction between the potentially binding substance(s) with CITED2, CDK11b^{p58} and/or splicing factor 3B subunit 1 (SF3B1) and/or 14-3-3γ, whereby any of the components can be independently of each other in a liquid phase, for example in solution, or in suspension or can be bound to a solid phase, for example, in the form of an essentially planar surface or in the form of particles, pearls or the like. In a preferred embodiment a multitude of different potentially binding substances are immobilized on a solid surface like, for example, on a compound library chip, and CITED2, CDK11b^{p58} and/or splicing factor 3B subunit 1 (SF3B1) and/or 14-3-3γ (or a functional part thereof) or the bound complex thereof are subsequently contacted with such a chip.

In the context of the present invention, the term "biological activity" shall preferably include the cellular functions of CITED2, CDK11b^{p58} and/or splicing factor 3B subunit 1 (SF3B1) and/or 14-3-3γ, the complex of CITED2 with CDK11b^{p5g} and/or splicing factor 3B subunit 1 (SF3B1) and/or 14-3-3γ, and/or the fragments thereof in the cell in the process of the mitotic cell cycle, for example their function(s) in the spindle assembly and/or in inducing other factors, necessary for proper centrosome segregation and subsequent for a proper spindle assembly.

In a second aspect of the present invention, the problem of the present invention is solved by providing a method for identifying a compound that modulates the mitotic cell cycle in cancer cells, by modulating at least one of the interaction of CITED2 with at least one molecule selected from the group consisting of CDK11b^{p58}, splicing factor 3B subunit 1 (SF3B1) and 14-3-3γ in a mammalian cell, comprising the steps of: a) contacting at least one of CITED2, CDK11b^{p58}, splicing factor 3B subunit 1 (SF3B1) and/or 14-3-3γ binding fragment of CITED2, a cell expressing CITED2 and/or a cell expressing CDK11b^{p58} and/or a cell expressing splicing factor 3B subunit 1 (SF3B1) and/or 14-3-3γ binding fragment of CITED2 with at least one compound that potentially modulates the interaction of CITED2 with at least one molecule selected from the group consisting of CDK11b^{p58}, splicing factor 3B subunit 1 (SF3B1) and 14-3-3γ, b) identifying a modulation of the interaction of CITED2 or the fragment thereof with at least one molecule selected from the group consisting of CDK1 1b^{p58}, splicing factor 3B subunit 1 (SF3B1) and 14-3-3γ in the presence of said at least one compound, compared to the absence of said at least one compound, and c) identifying at least one compound of step b) as modulating, in particular specifically modulating, the mitotic cell cycle in cancer cells based on the modulation as identified in step b).

Preferred is the method according to the present invention, wherein said modulation is selected from a decrease or an increase of said interaction, preferably an inhibition of the interaction.

Further preferred is the method according to the present invention, wherein the cancer cells are selected from colorectal cancer cells, gastric cancer cells, rectal cancer cells, breast cancer cells, cervical cancer cells, endocervical cancer cells, colon cancer cells, esophageal cancer cells, brain cancer cells, head and neck cancer cells, renal cancer cells, meningeal cancer cells, glioma, glioblastoma, lung cancer cells, mesothelioma, ovarian cancer cells, pancreatic cancer cells, neuroendocrine cancer cells, prostatic cancer cells, skin cancer cells, stomach cancer cells, thyroid cancer cells, uterine cancer cells, and testicular cancer cells, and are preferably selected from colorectal cancer cells.

Further preferred is the method according to the present invention, wherein the identifying in step c) comprises determining centrosome segregation and a subsequent bipolar spindle assembly in the mammalian cell, preferably determining of bipolar centrosome separation and/or the formation of chaotic monopolar mitotic spindles.

Further preferred is the method according to the present invention, wherein the identifying in step c) further comprises determining tumor volume and tumor number per subject.

Further preferred is the method according to the present invention, wherein said compound is selected from the group consisting of a peptide library, a combinatory library, a cell extract, a "small molecular drug", an antisense oligonucleotide, such as a gapmer antisense oligonucleotide, an siRNA, and an antibody, in particular an antibody against CITED2 or a fragment thereof, e.g. a F(ab)2-fragment, specifically interfering with the binding of CITED2 or the binding fragment thereof to at least one molecule selected from the group consisting of CDK11b^{p58}, splicing factor 3B subunit 1 (SF3B1) and 14-3-3γ and an antibody oligo conjugate (AOC) targeting CITED2 or antigen binding fragment oligo conjugate thereof. Antibody oligo conjugates and their uses are known to the person of skill and described in the art (e.g. Dugal-Tessier J, Thirumalairajan S, Jain N. Antibody-Oligonucleotide Conjugates: A Twist to Antibody-Drug Conjugates. J Clin Med. 2021 Feb 18;10(4):838. doi: 10.3390/jcm10040838. PMID: 33670689; PMCID: PMC7922418).

Further preferred is the method according to the present invention, further comprising a computational analysis and optimization of said compound based on the structure, in particular the three-dimensional and/or crystal structure of CITED2 and/or CDK11b^{p58} and/or splicing factor 3B subunit 1 (SF3B1) and/or 14-3-3γ.

The inventors found a previously unappreciated bimodal function for CITED2 to safeguard accurate epithelial cell mitosis. While it acts as a transcriptional co-activator to control critical gene expression during M phase and G2/M transition, it also provides important scaffold function required for correct CDK11b^{p58} localization during early centrosome maturation. CDK11 was shown to be required for Aurora A and Plk1 localization to centrosomes during early mitosis (31). Loss of CITED2 does not seem to impact the presence of these kinases at centrosomes but rather affects their activation suggesting that CITED2 is not only required for proper distribution of CDK11 onto MTOCs but also for its kinase function to promote downstream phosphorylation of other mitotic kinases, which take part in centrosome maturation and subsequent bipolar spindle formation. This essential requirement of CITED2 in mitotic fidelity explains the strong impact on actively cycling intestinal stem cells. In addition to orchestrating the priming of Aurora and Plk1 kinase activation during early mitosis (35), CDK11 plays a major role in transcriptional regulation and pre-mRNA splicing (36).

The inventors found that CITED2 also interacted with the recently identified CDK11 substrate splicing factor 3B subunit 1 (SF3B1) (37), a U2 small nuclear ribonucleoprotein that is phosphorylated during spliceosome activation. In line with this notion, the inventors observed significantly reduced alternative splicing events in *Cited2* KO samples (Ceteci and Greten, unpublished results).

In addition to SF3B1, the inventors also detected another CDK11 substrate 14-3-3γ as a potential CITED2-interacting protein. It is already known that CDK11 interacts with several 14-3-3 isoforms including 14-3-3γ (38). 14-3-3 adaptor proteins fulfill important scaffold function in a variety of cellular processes including cell cycle regulation, apoptosis, and transcriptional regulation (39). Particularly, 14-3-3γ was shown to localize to the centrosome and interacts with centrosomal proteins (40, 41). 14-3-3y-Plkl novel interaction pathway stimulates the catalytic activity of Plk1 and knockdown of 14-3-3γ leads to a prometaphase/metaphase-like arrest due to the activation of the spindle assembly checkpoint (42). Moreover, 14-3-3γ prevents centrosome amplification and spindle multi-polarity in HCT116 cells (41). As CITED2 protein reduction also mimics 14-3-3γ related mitotic phenotypes, there is an interest to investigate further if CITED2-14-3-3γ interaction plays a key role in regulation of early mitosis in terms of both scaffolding CITED2 binding to CDK11b as well as priming its correct localization to MTOCs in order to control centrosome maturation and function.

In the context of the present invention, the term "CDK11b^{p58}" shall be understood as indicating/representing the mammalian, in particular human or ortholog/homolog of the human p58 isoform of cyclin-dependent kinase 11B (CDK11b^{p58}) gene and/or protein and/or mRNA, in particular mouse. Also, the term shall comprise the complete CDK11b^{p58} polypeptide or fragment(s) of the CDK11b^{p58} polypeptide in different preparations, such as in the cellular context or purified from the cell. The human amino acid sequence of CDK11b^{p58} can be, e.g. found at UniProt P21127-12.

In the context of the present invention, the term " splicing factor 3B subunit 1 (SF3B1) and 14-3-3γ" shall be understood as the substrates of the p58 isoform of cyclin-dependent kinase 11B as obtained in the experiments as performed in the context of the present invention such as the splicing factor 3B subunit 1 (SF3B1) and 14-3-3γ. SF3B1 and 14-3-3γ indicate/represent the mammalian, in particular human or ortholog/homolog of the human SF3B1 and 14-3-3γ gene and/or protein and/or mRNA, in particular mouse. Also, the term shall comprise the complete SF3B1 and 14-3-3γ polypeptides or fragment(s) of SF3B1 and 14-3-3γ polypeptides in different preparations, such as in the cellular context or purified from the cell. The human amino acid sequences of SF3B1 and 14-3-3γ can be, e.g. found at UniProt O75533 and P61981, respectively.

In a third aspect of the present invention, the problem of the present invention is solved by providing a screening tool for screening for a compound that modulates the expression, the stability, the biological activity and/or the interaction of CITED2 with at least one molecule selected from the group consisting of CDK11b^{p58}, splicing factor 3B subunit 1 (SF3B1) and 14-3-3γ, comprising an isolated cell expressing CITED2, and/or expressing an CDK11b^{p5g} and/or splicing factor 3B subunit 1 (SF3B1) and/or 14-3-3γ binding fragment thereof, wherein said cell optionally expresses CDK11b^{p58} and/or an CITED2 binding fragment thereof, wherein preferably said CITED2 and/or CDK11b^{p58} and/or splicing factor 3B subunit 1 (SF3B1) and/or 14-3-3γ and/or the fragments thereof are immobilized and/or labeled.

In a fourth aspect of the present invention, the problem of the present invention is solved by providing a method for determining the level of proper centrosome segregation and subsequent bipolar spindle assembly in a sample obtained from a subject, comprising detecting the concentration of CITED2 in the sample, wherein a decrease of the concentration of CITED2 in the sample when compared to a control indicates an improper centrosome segregation and subsequent an increase of the formation of chaotic monopolar mitotic spindles in said sample.

Also provided is a method for determining the level of CITED2 and/or CDK11b^{p58} and/or splicing factor 3B subunit 1 (SF3B1) and/or 14-3-3γ in a sample obtained from a subject, comprising detecting of binding of CITED2 in the sample to at least one molecule selected from the group consisting of CDK11b^{p58}, splicing factor 3B subunit 1 (SF3B1) and 14-3-3γ, wherein a decrease of the binding, when compared to a control indicates an improper centrosome segregation and subsequent an increase of the formation of chaotic monopolar mitotic spindles in said sample.

In a fifth aspect of the present invention, the problem of the present invention is solved by providing a method for detecting the progression of cancer cells and/or the risk for the progression of cancer cells in a sample, comprising performing a method according to the present invention, wherein a decrease of CITED2 and/or decrease of binding of CITED2 to at least one molecule selected from the group consisting of CDK11b^{p58}, splicing factor 3B subunit 1 (SF3B1) and 14-3-3γ in the sample is indicative for the formation of chaotic monopolar mitotic spindles in said sample and a decrease of progression of cancer cells and/or the risk for the progression of cancer cells.

In a sixth aspect of the present invention, the problem of the present invention is solved by providing a method for manufacturing a pharmaceutical composition for treating or preventing a disease or condition selected from said cancer cells, comprising the steps of performing a method for identifying a compound according to the present invention, wherein said compound preferably is an CITED2 antisense oligonucleotide, such as a gapmer antisense oligonucleotide, an siRNA, or an antibody, in particular an antibody against CITED2 or fragment thereof, e.g. a F(ab)2-fragment, specifically interfering with the binding of CITED2 or the binding fragment thereof, or an AOC as described herein, to at least one molecule selected from the group consisting of CDK11b^{p58}, splicing factor 3B subunit 1 (SF3B1) and 14-3-3γ, and formulating said compound as identified into a pharmaceutical composition.

Preferably, said composition comprises an aqueous formulation. The term "pharmaceutical composition" refers to a preparation which is in such form as to permit the biological activity of an active ingredient contained therein (here, the at least one compound) to be effective, and which contains no additional components which are unacceptably toxic to a subject to which the composition would be administered. A pharmaceutical composition of the present invention can be administered by a variety of methods known in the art. As will be appreciated by the skilled artisan, the route and/or mode of administration will vary depending upon the desired results. Pharmaceutically acceptable diluents include saline and aqueous buffer solutions. A "pharmaceutically acceptable carrier" refers to an ingredient in a pharmaceutical formulation, other than an active ingredient, which is nontoxic to a subject. Pharmaceutically acceptable carriers include any and all solvents, dispersion media, coatings, antibacterial and antifungal agents, isotonic and absorption delaying agents, and the like that are physiologically compatible. The carrier can be suitable for intravenous, intramuscular, subcutaneous, parenteral, spinal or epidermal administration (e.g., by injection or infusion). Preferred is the pharmaceutical composition according to the present invention, wherein the composition is for injection, oral and/or nasal application.

The pharmaceutical compositions according to the present invention may also contain adjuvants such as preservatives, wetting agents, emulsifying agents and dispersing agents. Prevention of presence of microorganisms may be ensured both by sterilization procedures and by the inclusion of various antibacterial and antifungal agents, for example, paraben, chlorobutanol, phenol, sorbic acid, and the like. It may also be desirable to include isotonic agents, such as sugars, sodium chloride, and the like into the compositions. In addition, prolonged absorption of the injectable pharmaceutical form may be brought about by the inclusion of agents which delay absorption such as aluminum monostearate and gelatin.

The pharmaceutical compositions according to the present invention may be in liquid, dry or semi-solid form, such as, for example, in the form of a tablet, coated tablet, effervescent tablet, capsule, powder, granulate, sugar-coated tablet, lozenge, pill, ampoule, drop, suppository, emulsion, ointment, gel, tincture, paste, cream, moist compress, gargling solution, plant juice, nasal agent, inhalation mixture, aerosol, mouthwash, mouth spray, nose spray, or room spray. Preferred is an injectable composition.

Administration of an agent, e.g., a compound, optionally in the form of a pharmaceutical composition, can be accomplished by any method which allows the agent to reach the target location, e.g. in a cell or blood. These methods include, e.g., injection, deposition, implantation, suppositories, oral ingestion, inhalation, topical administration, or any other method of administration where access to the target cells by the agent is obtained. Injections can be, e.g., intravenous, intradermal, subcutaneous, intramuscular or intraperitoneal. Implantation includes inserting implantable drug delivery systems, e.g., microspheres, hydrogels, polymeric reservoirs, cholesterol matrices, polymeric systems, e.g., matrix erosion and/or diffusion systems and non-polymeric systems, e.g., compressed, fused or partially fused pellets. Suppositories include glycerin suppositories. Oral ingestion doses can be enterically coated. Inhalation includes administering the agent with an aerosol in an inhalator, either alone or attached to a carrier that can be absorbed. The agent can be suspended in liquid, e.g., in dissolved or colloidal form. The liquid can be a solvent, partial solvent or non-solvent. In many cases, water or an organic liquid can be used.

Further provided is a pharmaceutical composition as produced according to the present invention, or a compound as identified with a method according to the present invention, preferably an CITED2 antisense oligonucleotide, such as a gapmer antisense oligonucleotide, an siRNA, or an antibody, in particular an antibody against CITED2 or fragment thereof, e.g. a F(ab)2-fragment, specifically interfering with the binding of CITED2 or the binding fragment thereof to at least one molecule selected from the group consisting of CDK11b^{p58}, splicing factor 3B subunit 1 (SF3B1) and 14-3-3γ for use in the prevention or treatment of a disease or condition caused by said cancer cells, such as, for example, selected from gastric cancer cells, colon cancer cells, pancreatic, lung, and/or rectal cancer cells, wherein preferably the prevention or treatment is in combination with at least one checkpoint-inhibitor (ICI). ICI is described in the literature and well known to the person of skill (see, for example, Wu M, Huang Q, Xie Y, Wu X, Ma H, Zhang Y, Xia Y. Improvement of the anticancer efficacy of PD-1/PD-L1 blockade via combination therapy and PD-L1 regulation. J Hematol Oncol. 2022 Mar 12;15(1):24. doi: 10.1186/s13045-022-01242-2. PMID: 35279217; PMCID: PMC8917703).

Preferred is the pharmaceutical composition for use according to the present invention, wherein the composition is for injection, oral and/or nasal application.

In a further embodiment of the method according to the present invention, the interacting compound identified as outlined above, which may or may not have gone through additional rounds of modification and selection, is admixed with suitable auxiliary substances and/or additives. Such substances comprise pharmacological acceptable substances, which increase the stability, solubility, biocompatibility, or biological half-life of the interacting compound or comprise substances or materials, which have to be included for certain routs of application like, for example, intravenous solution, sprays, band-aids or pills. Carriers, excipients and strategies to formulate a pharmaceutical composition, for example to be administered systemically or topically, by any conventional route, in particular enterally, e.g. orally, e.g. in the form of tablets or capsules, parenterally, e.g. in the form of injectable solutions or suspensions, topically, e.g. in the form of lotions, gels, ointments or creams, or in nasal or a suppository form are well known to the person of skill and described in the respective literature.

In general, the attending physician will base a treatment on the compound as identified, and optionally also on other individual patient data (clinical data, family history, DNA, etc.), and a treatment can also be performed based on the combination of these factors. This method of the present invention for example involves integrating individual diagnostic blood or other data with patient clinical information and general healthcare statistics to enable, for example, the application of personalized medicine to the patient. Significant information about drug effectiveness, drug interactions, and other patient status conditions can be used, too.

Preferably, an active agent is administered in form of a pharmaceutical composition as described herein, such as an antibody, nucleotide or an inhibiting binding compound for the CITED2 and/or CDK11b^{p58} and/or splicing factor 3B subunit 1 (SF3B1) and/or 14-3-3γ binding. The CDK11b^{p58} and/or splicing factor 3B subunit 1 (SF3B1) and/or 14-3-3γ or CITED2 or complex binding compound or AOC as described herein according to the present invention, and optional other therapeutic agents may be administered simultaneously, sequentially or simultaneously. Preferably the prevention or treatment is in combination with at least one checkpoint-inhibitor (ICI).

Further provided is a method for preventing or treating a disease or condition caused by cancer, such as, for example, selected from gastric cancer, colon cancer, and/or rectal cancer in a subject in need of such prevention and/or treatment, comprising administering to said subject an effective amount of the pharmaceutical composition as produced according to the present invention, or a compound as identified with a method according to the present invention, preferably an CITED2 antisense oligonucleotide, such as a gapmer antisense oligonucleotide, an siRNA, or an antibody, in particular an antibody against CITED2 or fragment thereof, e.g. a F(ab)2-fragment, specifically interfering with the binding of CITED2 or the binding fragment thereof to at least one molecule selected from the group consisting of CDK11b^{p58}, splicing factor 3B subunit 1 (SF3B1), 14-3-3γ and AOC or binding fragment thereof as described herein.

In a seventh aspect of the present invention, the problem of the present invention is solved by providing a method for monitoring the treatment of a disease or condition caused by said cancer cells, such as, for example, selected from gastric cancer cells, colon cancer cells, and/or rectal cancer cells in a subject, comprising performing the method according to the present invention on a sample obtained from a subject before and after or during a treatment according to the present invention, and monitoring the treatment of the disease or condition based on the differences as detected between the samples.

Based on the monitoring, the attending physician can conclude on the success or progress of the treatment or prevention and adjust a further treatment or prevention accordingly. Monitoring may be repeated during the course of a therapy or for a prevention.

Decreased CITED2 expression was reported in ageing tendon-derived stem/progenitor cells, which was associated with decreased mitotic cell cycle progression, while CITED2 overexpression potentiated their abilities to regenerate damaged tissues through downregulation of p21, and upregulation of c-MYC (17, 43). Tissue regeneration and cancer is often linked as they require high rates of cycling stem cells. It is tempting to postulate that in particular circumstances, an elevated CITED2 expression might contribute to uncontrolled self-renewal and proliferation of stem cells and help to originate CSC. In line with this hypothesis, elevated CITED2 expression has been found in CD34⁺ leukemic cells compared to normal cells (44).

As CITED2 is critical for cells during mitosis only and considering that the impaired CITED2/CDK11bp58 interaction mimics a pan-aurora/PLK1/Eg5 inhibition, the inventors reasoned that CITED2 ablation may represent a promising strategy to target cancer. Indeed, loss of CITED2 in all adult tissues of mice was well-tolerated and demonstrated clinically relevant tumor shrinkages in the intestine pointing out a therapeutic window for human CRC. Because the concomitant DNA-damage induced cGAS/STING dependent activation of IFN signaling, it induces a CD8⁺ mediated anti-tumor immunity and sensitization to checkpoint therapy even suboptimal inhibition of CITED2 may lead to immune mediated eradication of tumor cells that escape cell cycle inhibition.

Thus, a method for identifying a compound that modulates the mitotic cell cycle in cancer cells by modulating at least one of the expression, the amount, the stability and/or the biological activity of the protein CITED2 and/or CITED2 containing protein complexes in a mammalian cell was designed.

The biological activity concerned is preferably selected from the interaction of CITED2 with at least one molecule selected from the group consisting of CDK11b^{p58}, splicing factor 3B subunit 1 (SF3B1) and 14-3-3γ.

In a second aspect, a more specific method for identifying a compound that modulates the mitotic cell cycle in cancer cells, by modulating at least one of the interaction of CITED2 with at least one molecule selected from the group consisting of CDK11b^{p58}, splicing factor 3B subunit 1 (SF3B1) and 14-3-3γ in a mammalian cell was designed.

The methods comprise the step of contacting CITED2 or a functional fragment thereof and/or a cell expressing CITED2 or a functional fragment thereof with at least one compound that potentially modulates at least one of the expression, the amount, the stability and/or the biological activity of the protein CITED2 in a mammalian cell. Identifying a modulation of at least one of the expression, the amount, the stability and/or the biological activity of the protein CITED2 in the presence of said at least one compound then identifies a compound as specifically modulating mitotic cell cycle in cancer cells. In this general method, preferably the biological activity is selected from the interaction of CITED2 with at least one molecule selected from the group consisting of CDK11b^{p58}, splicing factor 3B subunit 1 (SF3B1) and 14-3-3γ.

For identifying a compound that modulates the mitotic cell cycle in cancer cells, by modulating at least one of the interaction of CITED2 with at least one molecule selected from the group consisting of CDK11b^{p58}, splicing factor 3B subunit 1 (SF3B1) and 14-3-3γ in a mammalian cell, comprising the steps of: a) contacting at least one of CITED2, CDK11b^{p58}, splicing factor 3B subunit 1 (SF3B1) and/or 14-3-3γ binding fragment of CITED2, a cell expressing CITED2 and/or a cell expressing CDK11b^{p58} and/or a cell expressing splicing factor 3B subunit 1 (SF3B1) and/or 14-3-3γ binding fragment of CITED2 with at least one compound that potentially modulates the interaction of CITED2 with at least one molecule selected from the group consisting of CDK11b^{p58}, splicing factor 3B subunit 1 (SF3B1) and 14-3-3γ.

This first step relates to a binding of the molecule to the targets.

In the next step, b) a modulation of the interaction of CITED2 or the fragment thereof with at least one molecule selected from the group consisting of CDK11b^{p58}, splicing factor 3B subunit 1 (SF3B1) and 14-3-3γ in the presence of said at least one compound is detected. The modulation is selected from a) an increase of said expression, the amount, biological activity and/or interaction (binding), and/or b) a stabilization of said expression, the amount, biological activity and/or interaction (binding), and c) a decrease of the expression, the amount, the biological activity and/or the interaction (binding) of CITED2, of CDK11b^{p58} and/or of splicing factor 3B subunit 1 (SF3B1) and/or 14-3-3γ with each other in the sample or a cell. Preferred is an inhibition of the expression, the amount, the biological activity and/or the interaction (binding) of CITED2, of CDK11b^{p58} and/or of splicing factor 3B subunit 1 (SF3B1) and/or of 14-3-3γ with each other in the sample or a cell. Specifically targeted is the binding between CITED2 with at least one molecule selected from the group consisting of CDK11b^{p58}, splicing factor 3B subunit 1 (SF3B1) and 14-3-3γ or binding fragments thereof.

In a next step, a compound as identified in step b) is further identified as specifically modulating mitotic cell cycle in cancer cells in the subject (see, for example, experiments and figures). Further preferred is the method according to the present invention, further comprising testing said compound as identified for its activity to induce or reduce mitotic cell cycle, in particular in the context of the progression of cancer cells, for example by detecting tumor volume and tumor number per subject. Respective assays are also known to the person of skill, and can be taken from the respective literature.

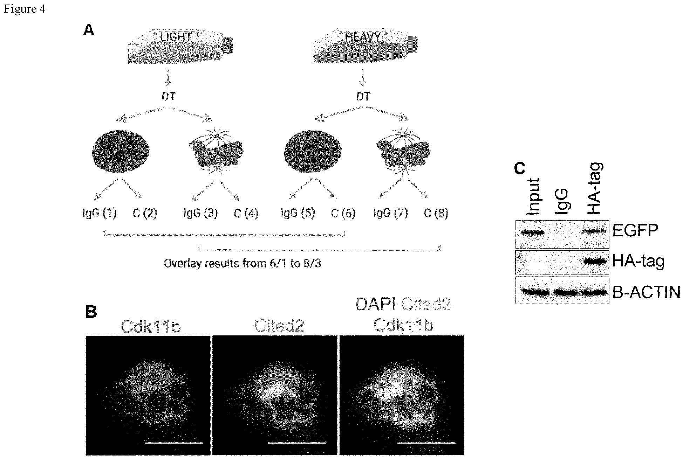

Prompted by the unexpected localization of CITED2 at centrosomes, the inventors reasoned that CITED2 may impact centrosome maturation independently of its well-known function as a transcriptional co-activator. Therefore, the inventors performed mass spectrometry analysis to identify putative CITED2 binding proteins known to be essential for mitotic fidelity. The inventors discovered several candidates specifically binding to CITED2 during G2/M phase. The inventors focused on cyclin-dependent kinase 11b (Cdk11b) as it is required for centrosome maturation and bipolar spindle formation (31). *Cdk11b* encodes a 110-kDa protein (Cdk11b^{p11a0}) throughout the cell cycle and a 58-kDa protein (Cdk11b^{p58}) that is specifically translated from an internal ribosome entry site sequence during G2/M (32, 33). Confocal microscopy indicated colocalization of CITED2 and Cdk11b during mitosis and immunoblot analysis confirmed binding of CITED2 to Cdk11b^{p58} in Cdk11b^{p58}-overexpressing Mode-K cells (Figures 4B and 4C).

It was surprisingly found in the context of the present invention, that CITED2-driven Cdk11b^{p58} expression mediates Aurora-PLK1-EG5 key signaling cascade in the regulation of early mitosis to control centrosome maturation and bipolar spindle assembly, which finally contribute to chromosome stability and faithful cell division. An inhibition of the interaction of CITED2 with Cdk11b^{p58} caused an improper centrosome segregation and subsequent an increase of the formation of chaotic monopolar mitotic spindles leading to less progression of cancer cells. Preferred is therefore a method according to the present invention, wherein the identifying in step c) comprises determining the mitotic cell cycle in cancer cells (i.e. compared to a non-diseased control) and/or a modulation of the formation of chaotic monopolar mitotic spindles in cancer cells.

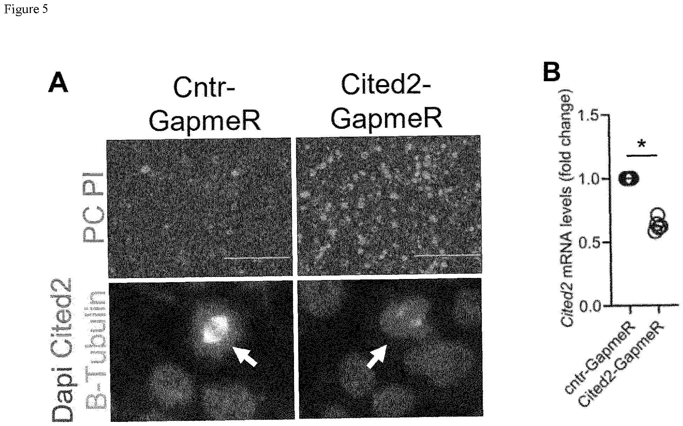

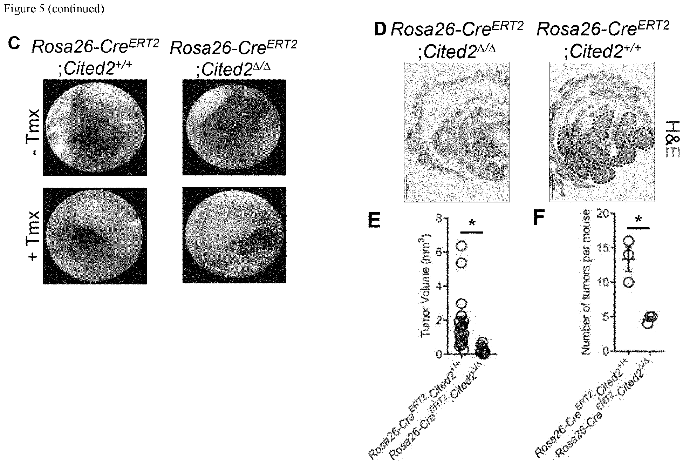

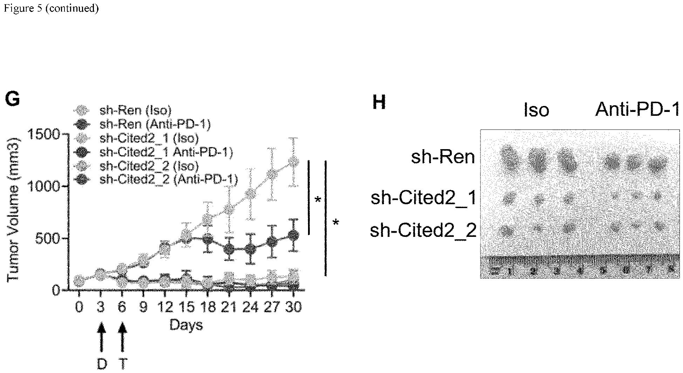

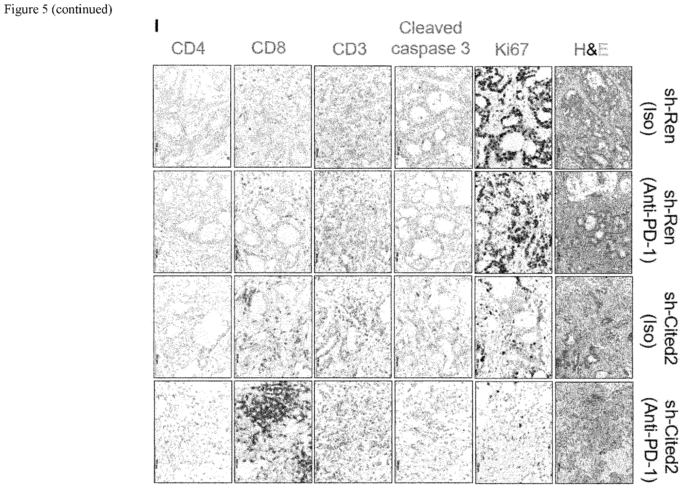

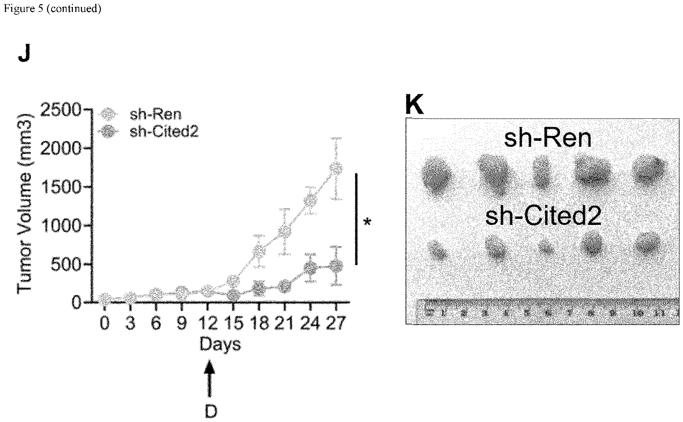

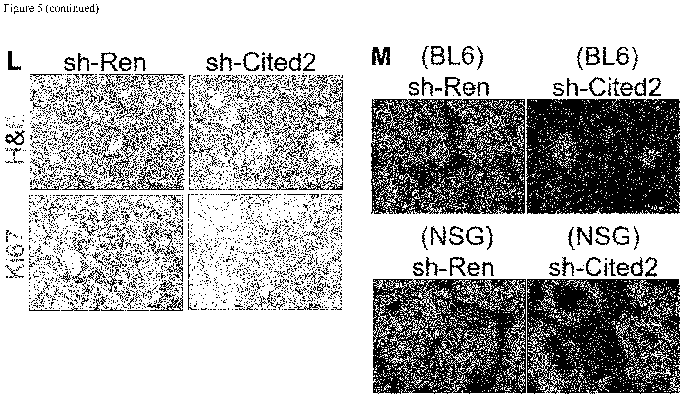

Further preferably, said identifying comprises a method selected from dual immunofluorescence staining, qRT-PCR, representative endoscopy images of colonic tumors in AOM-DSS-treated mice from indicated genotypes, immunoprecipitation and measuring the induction or reduction of the formation of chaotic monopolar mitotic spindles, for example by detecting collapsed bipolar spindle formation in SW48 cells following CITED2 inhibition with antisense gapmer treatment. Said identifying also comprises quantification of colonic tumor volume and number in mice (Figure 5).

More preferred is a method according to the present invention, wherein said compound is selected from the group consisting of a peptide library, a combinatory library, a cell extract, a "small molecular drug", an antisense oligonucleotide such as a gapmer antisense oligonucleotide, an siRNA, and an antibody, in particular an antibody against CITED2 or fragment thereof specifically interfering with the binding of CITED2 to at least one molecule selected from the group consisting of CDK11b^{p58}, splicing factor 3B subunit 1 (SF3B1) and 14-3-3γ.

Particularly preferred are antibody fragments or siRNA molecules against CITED2 that reduce mitotic cell cycle in cancer cells. The inventors conclude that these F(ab)2-Fragments prevent binding of CITED2 to at least one molecule selected from the group consisting of CDK11b^{p58}, splicing factor 3B subunit 1 (SF3B1) and 14-3-3γ and therefore leads to improper centrosome segregation. Consequently, monoclonal antibodies against CITED2 are also a promising therapeutic approach.

In the context of the present invention, a "small molecular drug" or "small molecule" is one that has a molecular weight of below 2000 Da, preferably of below 1000 Da, optimally in the range of between 600 and 200 Da.

Preferred is a method according to the present invention, wherein said CITED2 and/or said CDK11b^{p58} and/or splicing factor 3B subunit 1 (SF3B1) and/or 14-3-3γ is/are immobilized, such as, for example, on a microtiter plate, beads or a chip, like a glass slide.

Further preferred is a method according to the present invention, wherein said CITED2 and/or CDK11b^{p58} and/or splicing factor 3B subunit 1 (SF3B1) and/or 14-3-3γ and/or the fragments thereof are suitably labeled. Measuring of binding of the compound to CITED2 and/or CDK11b^{p58} and/or splicing factor 3B subunit 1 (SF3B1) and/or 14-3-3γ and/or the fragments thereof can be carried out either by measuring a marker that can be attached either to the protein or to the potentially interacting compound. Suitable markers are known to someone of skill in the art and comprise, for example, fluorescence or radioactive markers. The binding of the two components can, however, also be measured by the change of an electrochemical parameter of the binding compound or of the protein, e.g. a change of the redox properties of CITED2 and/or CDK11b^{p58} and/or splicing factor 3B subunit 1 (SF3B1) and/or 14-3-3γ and/or the fragments thereof or the binding compound, upon binding. Suitable methods of detecting such changes comprise, for example, potentiometric methods. Further methods for detecting and/or measuring the binding of the two components to each other are known in the art and can without limitation also be used to measure the binding of the potential interacting compound to CITED2 and/or CDK11b^{p58} and/or splicing factor 3B subunit 1 (SF3B1) and/or 14-3-3γ and/or the fragments thereof. The effect of the binding of the compound or the activity of CITED2 and/or CDK11b^{p58} and/or splicing factor 3B subunit 1 (SF3B1) and/or 14-3-3γ and/or the fragments thereof can also be measured indirectly, for example, by assaying an enzymatic activity of CITED2 and/or CDK11b^{p58} and/or splicing factor 3B subunit 1 (SF3B1) and/or 14-3-3γ and/or the fragments thereof after binding.

Even further preferred is a method according to the present invention that further comprises a computational analysis and optimization of the compound based on the structure, in particular the three-dimensional and/or crystal structure of CITED2 and/or CDK11b^{p58} and/or splicing factor 3B subunit 1 (SF3B1) and/or 14-3-3γ.

According to the invention, said cell is selected from the group of recombinant host cells expressing CITED2 and/or CDK11b^{p58} and/or splicing factor 3B subunit 1 (SF3B1) and/or 14-3-3γ or binding fragment thereof, wherein said recombinant host cells optionally express CITED2 and/or CDK11b^{p58} and/or splicing factor 3B subunit 1 (SF3B1) and/or 14-3-3γ or binding fragment thereof. The cells may be mammalian, such as human cell, yeast cells; and (recombinant) bacterial cells.

As a further step after measuring the binding of a potentially interacting compound and after having measured at least two different potentially interacting compounds at least one compound can be selected, for example, on grounds of the measured binding activity or on grounds of the detected increase of CITED2 and/or CDK11b^{p58} and/or splicing factor 3B subunit 1 (SF3B1) and/or 14-3-3γ (binding) activity and/or inhibition.

The thus selected binding compound can then in a preferred embodiment modified in a further step. Modification can be effected by a variety of methods known in the art, which include without limitation the introduction of novel side chains or the exchange of functional groups like, for example, introduction of halogens, in particular F, Cl or Br, the introduction of lower alkyl groups, preferably having one to five carbon atoms like, for example, methyl, ethyl, n-propyl, isopropyl, n-butyl, isobutyl, tert-butyl, n-pentyl or iso-pentyl groups, lower alkenyl groups, preferably having two to five carbon atoms, lower alkynyl groups, preferably having two to five carbon atoms or through the introduction of, for example, a group selected from the group consisting of NH₂, NO₂, OH, SH, NH, CN, aryl, heteroaryl or COOH group.

The thus modified binding substances are than individually tested with a method of the present invention, i.e. they are contacted with CITED2, and/or CDK11b^{p58} and/or splicing factor 3B subunit 1 (SF3B1) and/or 14-3-3γ and/or the fragments thereof and subsequently binding of the modified compounds to the CITED2 and/or CDK11b^{p58} and/or splicing factor 3B subunit 1 (SF3B1) and/or 14-3-3γ and/or the fragments thereof is measured. In this step, both the binding per se can be measured and/or the effect of the function of the CITED2 and/or CDK11b^{p58} and/or splicing factor 3B subunit 1 (SF3B1) and/or 14-3-3γ and/or the fragments thereof like, e.g. the binding to CITED2 and/or CDK11b^{p58} and/or splicing factor 3B subunit 1 (SF3B1) and/or 14-3-3γ and/or the fragments thereof and/or the biological activity of the polypeptide(s) can be measured. If needed the steps of selecting the binding compound, modifying the binding compound, contacting the binding compound with CITED2 and/or CDK11b^{p58} and/or splicing factor 3B subunit 1 (SF3B1) and/or 14-3-3γ and/or the fragments thereof and measuring the binding of the modified compounds to the protein(s) can be repeated a third or any given number of times as required. The above described method is also termed "directed evolution" since it involves a multitude of steps including modification and selection, whereby binding compounds are selected in an "evolutionary" process optimizing its capabilities with respect to a particular property, e.g. its binding activity, its ability to activate or modulate, such as inhibit, the activity of the polypeptide(s) of CITED2 and/ CDK1 1b^{p58} and/or splicing factor 3B subunit 1 (SF3B1) and/or 14-3-3γ and/or the fragments thereof.

According to another aspect thereof, the object of the present invention is solved by providing a screening tool for screening for a compound that modulates the expression, the stability, the biological activity and/or the interaction of CITED2 with CDK11b^{p58} and/or splicing factor 3B subunit 1 (SF3B1) and/or 14-3-3γ, comprising an isolated cell expressing CITED2, and/or expressing an CDK11b^{p58} and/or splicing factor 3B subunit 1 (SF3B1) and/or 14-3-3γ binding fragment thereof, wherein said cell optionally expresses CITED2 and/or CDK11b^{P58} binding fragment thereof.

The cell can be a prokaryotic or eukaryotic cell, and any genetic expression constructs can be present extrachromosomally or integrated into the chromosome. The polypeptides can be expressed in the form of a fusion protein, for example together with an enzymatically active moiety as reporter- construct, in order to be able to detect the expression product. Preferred host cells are derived from cells selected from the skeletal muscle, liver, adipose tissue, heart, pancreas, kidney, breast tissue, ovarian tissue, and/or hypothalamus. Thus, preferred is a screening tool according to the present invention, wherein said cell is selected from necrotic cells, cancer cells; recombinant host cells expressing said CITED2 and/or CDK11b^{P58} and/or splicing factor 3B subunit 1 (SF3B1) and/or 14-3-3γ binding fragments thereof; preferably mammalian, such as human cell, yeast cells; and (recombinant) bacterial cells.

In the context of the present invention, a "sample" can be any suitable biological sample obtained from a mammal suspected of comprising the compounds or targets (e.g. the proteins CITED2 and/or CDK11b^{p58} and/or splicing factor 3B subunit 1 (SF3B1) and/or 14-3-3γ). A preferred sample is a tumor tissue sample, in particular a human tumor tissue sample, suffering from cancer. Furthermore, a sample can be produced, such as a cell culture, cellular preparation or isolated or partially isolated compounds or targets as produced or collected. The sample may also comprise compounds to be screened as disclosed herein.

In the context of the present invention, a "subject" relates to a mammal, animal or individual, such as a person or patient suffering from cancer. In the context of the present invention, a "mammal" relates to a rodent, cat, dog, sheep, goat, horse, cattle, monkey or human, preferably a human suffering from cancer.

In the context of the present invention, the term "treatment" or "therapy" shall mean the attempted remediation of a health problem as disclosed herein, i.e. cancer in the subject.

In summary, the presented data identified a critical transcriptional co-activator independent function for CITED2 in governing genomic integrity and survival in cycling adult stem cells and suggest CITED2 as a critical component of a multiprotein complex that is required for CDK1 1b^{p58} function and to interact with its substrates. These highlight the clinical potential to target CITED2/CDK11b^{p58} interaction for cancer therapy.

The present invention relates to the following items:
Item 1. A method for identifying a compound that modulates the mitotic cell cycle in cancer cells by modulating at least one of the expression, the amount, the stability and/or the biological activity of the protein CITED2 and/or CITED2 containing protein complexes in a mammalian cell, comprising the steps of: a) contacting at least one of CITED2 or a functional fragment thereof and/or a cell expressing CITED2 or the functional fragment thereof with at least one compound that potentially modulates at least one of the expression, the amount, the stability and/or the biological activity of the protein CITED2 in the mammalian cell, b) identifying a modulation of at least one of the expression, the stability and/or the biological activity of the protein CITED2 in the presence of the at least one compound, when compared to the absence of said at least one compound, and c) identifying a compound of step b) as modulating, in particular as specifically modulating, the mitotic cell cycle in the cancer cells based on said modulation as identified in step b).
Item 2. The method according to item 1, wherein said modulation is selected from a decrease or an increase of the expression, the amount, the stability and/or the biological activity of the protein CITED2 in a mammalian cell, preferably an inhibition of the expression, the amount, the stability and/or the biological activity of the protein CITED2, wherein the biological activity is preferably selected from the interaction of CITED2 with at least one molecule selected from the group consisting of CDK11b^{p58}, splicing factor 3B subunit 1 (SF3B1), and 14-3-3γ.
Item 3. A method for identifying a compound that modulates the mitotic cell cycle in cancer cells, by modulating at least one of the interaction of CITED2 with at least one molecule selected from the group consisting of CDK11b^{p58}, splicing factor 3B subunit 1 (SF3B1), and 14-3-3γ in a mammalian cell, comprising the steps of: a) contacting at least one of CITED2, CDK11b^{p58}, splicing factor 3B subunit 1 (SF3B1) and/or 14-3-3γ binding fragment of CITED2, a cell expressing CITED2 and/or a cell expressing CDK1 1b^{p58} and/or a cell expressing splicing factor 3B subunit 1 (SF3B1), and/or 14-3-3γ binding fragment of CITED2 with at least one compound that potentially modulates the interaction of CITED2 with at least one molecule selected from the group consisting of CDK11b^{p58}, splicing factor 3B subunit 1 (SF3B1) and 14-3-3γ, b) identifying a modulation of the interaction of CITED2 or the fragment thereof with at least one molecule selected from the group consisting of CDK1 1b^{p58}, splicing factor 3B subunit 1 (SF3B1), and 14-3-3γ in the presence of said at least one compound, compared to the absence of said at least one compound, and c) identifying at least one compound of step b) as modulating, in particular specifically modulating, the mitotic cell cycle in cancer cells based on the modulation as identified in step b).
Item 4. The method according to item 3, wherein said modulation is selected from a decrease or an increase of said interaction, preferably an inhibition of the interaction.
Item 5. The method according to any one of items 1 to 4, wherein the cancer cells are selected from colorectal cancer cells, gastric cancer cells, rectal cancer cells, breast cancer cells, cervical cancer cells, endocervical cancer cells, colon cancer cells, esophageal cancer cells, brain cancer cells, head and neck cancer cells, renal cancer cells, meningeal cancer cells, glioma, glioblastoma, lung cancer cells, mesothelioma, ovarian cancer cells, pancreatic cancer cells, neuroendocrine cancer cells, prostatic cancer cells, skin cancer cells, stomach cancer cells, thyroid cancer cells, uterine cancer cells, and testicular cancer cells, and are preferably selected from colorectal cancer cells.
Item 6. The method according to any one of items 1 to 5, wherein the identifying in step c) comprises determining of centrosome segregation and subsequent bipolar spindle assembly in the mammalian cell, preferably determining bipolar centrosome separation and/or the formation of chaotic monopolar mitotic spindles.
Item 7. The method according to any one of items 1 to 6, wherein the identifying in step c) further comprises determining tumor volume and/or tumor number per subject.
Item 8. The method according to any one of items 1 to 7, wherein said compound is selected from the group consisting of a peptide library, a combinatory library, a cell extract, a "small molecular drug", an antisense oligonucleotide, such as a gapmer antisense oligonucleotide, an siRNA, and an antibody, in particular an antibody against CITED2 or a fragment thereof, e.g. a F(ab)2-fragment, specifically interfering with the binding of CITED2 or the binding fragment thereof to at least one molecule selected from the group consisting of CDK11b^{p58}, splicing factor 3B subunit 1 (SF3B1), 14-3-3γ, and an antibody oligo conjugate (AOC) targeting CITED2 or antigen binding fragment oligo conjugate thereof.
Item 9. The method according to any one of items 1 to 8, further comprising a computational analysis and optimization of said compound based on the structure, in particular the three-dimensional and/or crystal structure of CITED2 and/or CDK11b^{p58} and/or splicing factor 3B subunit 1 (SF3B1), and/or 14-3-3γ.
Item 10. A screening tool for screening a compound that modulates the expression, the stability, the biological activity and/or the interaction of CITED2 with at least one molecule selected from the group consisting of CDK11b^{p58}, splicing factor 3B subunit 1 (SF3B1), and 14-3-3γ, comprising an isolated cell expressing, in particular recombinantly expressing, CITED2, and/or expressing an CDK11b^{p58} and/or splicing factor 3B subunit 1 (SF3B1) and/or 14-3-3γ binding fragment thereof, wherein said cell optionally expresses CDK11b^{p58} and/or an CITED2 binding fragment thereof, wherein preferably said CITED2 and/or CDK11b^{p58} and/or splicing factor 3B subunit 1 (SF3B1) and/or 14-3-3γ and/or the fragments thereof are immobilized and/or labeled.
Item 11. A method for determining the level of proper centrosome segregation and subsequent bipolar spindle assembly in a sample obtained from a subject, comprising detecting the concentration of CITED2 in the sample, wherein a decrease of the concentration of CITED2 in the sample when compared to a control indicates an improper centrosome segregation and subsequent an increase of the formation of chaotic monopolar mitotic spindles in said sample.
Item 12. A method for determining the level of CITED2 and/or CDK11b^{p58} and/or splicing factor 3B subunit 1 (SF3B1) and/or 14-3-3γ in a sample obtained from a subject, comprising detecting of binding CITED2 in the sample to at least one molecule selected from the group consisting of CDK1 1b^{p58}, splicing factor 3B subunit 1 (SF3B1) and 14-3-3γ, wherein a decrease of the binding, when compared to a control indicates an improper centrosome segregation and a subsequent increase of the formation of chaotic monopolar mitotic spindles in said sample.
Item 13. A method for detecting the progression of cancer cells and/or the risk for the progression of cancer cells in a sample, comprising performing a method according to item 11 or 12, wherein a decrease of CITED2 and/or decrease of binding of CITED2 to at least one molecule selected from the group consisting of CDK11b^{p58}, splicing factor 3B subunit 1 (SF3B1) and 14-3-3γ in the sample is indicative for the formation of chaotic monopolar mitotic spindles in said sample and a decrease of the progression of cancer cells and/or the risk for the progression of cancer cells.
Item 14. A method for manufacturing a pharmaceutical composition for treating or preventing cancer cells, comprising the step of performing the method according to any of items 1 to 8, and formulating said compound as identified into a pharmaceutical composition, wherein said compound preferably is an CITED2 antisense oligonucleotide, such as a gapmer antisense oligonucleotide, an siRNA, or an antibody, in particular an antibody against CITED2 or fragment thereof, e.g. a F(ab)2-fragment, specifically interfering with the binding of CITED2 or the binding fragment thereof to at least one molecule selected from the group consisting of CDK11b^{p58}, splicing factor 3B subunit 1 (SF3B1), and 14-3-3γ.
Item 15. A pharmaceutical composition as produced according to item 14, or a compound as identified with a method according to any of items 1 to 8, preferably an CITED2 antisense oligonucleotide, such as a gapmer antisense oligonucleotide, an siRNA, or an antibody, in particular an antibody against CITED2 or fragment thereof, e.g. a F(ab)2-fragment, specifically interfering with the binding of CITED2 or the binding fragment thereof to at least one molecule selected from the group consisting of CDK11b^{p58}, splicing factor 3B subunit 1 (SF3B1) and 14-3-3γ for use in the prevention or treatment of a disease or condition caused by said cancer cells, such as, for example, selected from gastric cancer cells, colon cancer cells, and/or rectal cancer cells.
Item 16. A method for monitoring the treatment of a disease or condition caused by cancer cells, such as, for example, gastric cancer cells, colon cancer cells, and/or rectal cancer cells in a subject, comprising performing the method according to item 11 or 12 on a sample obtained from a subject before and after or during a treatment according to item 15, and monitoring the treatment of the disease or condition based on the differences as detected between the samples.

In the accompanying figures and the accompanying sequence listing,

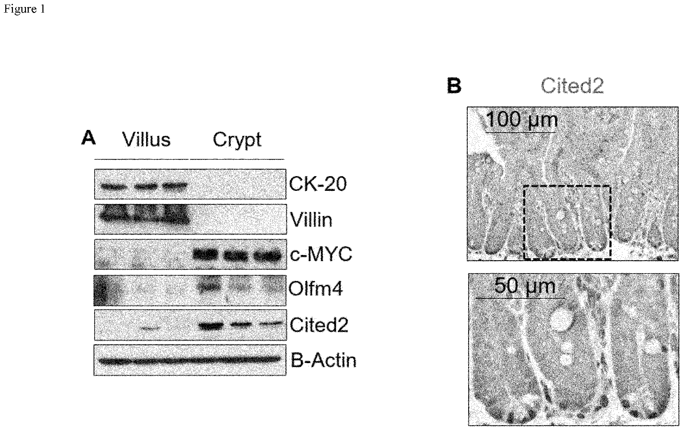

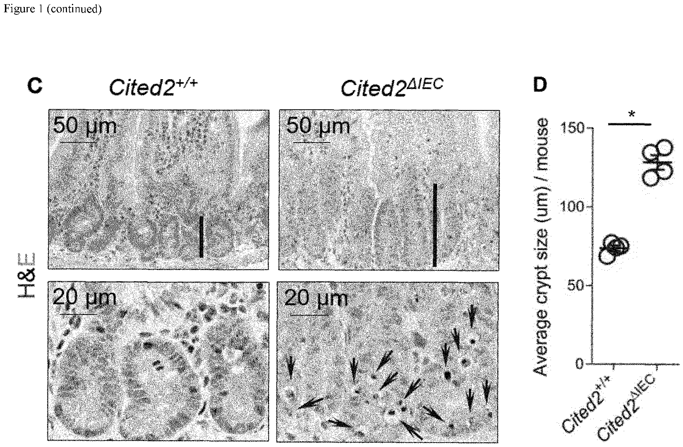

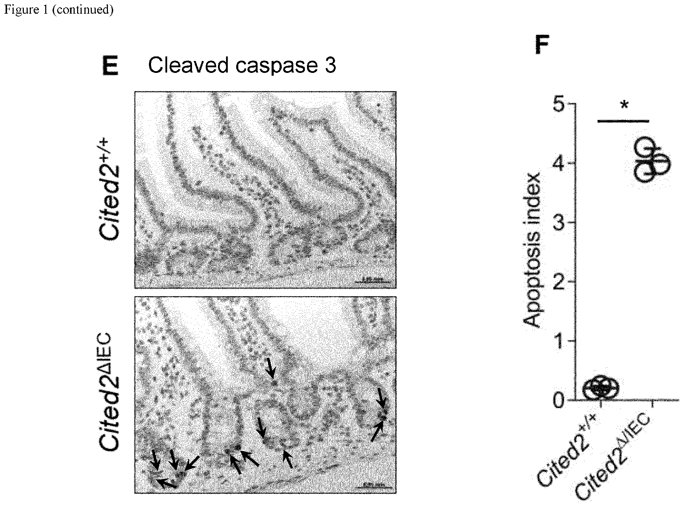

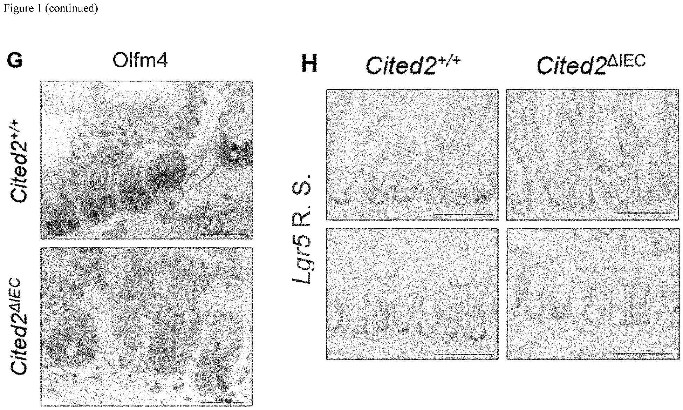

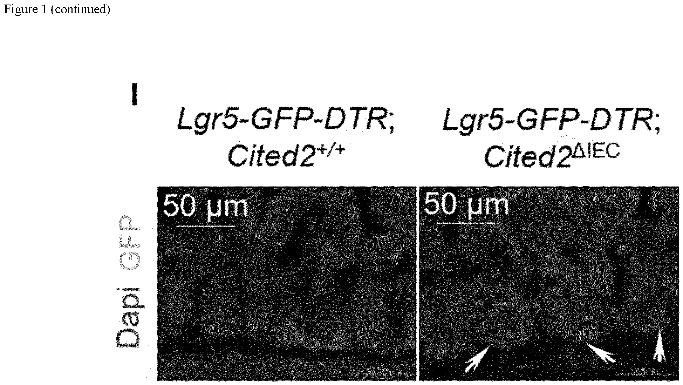

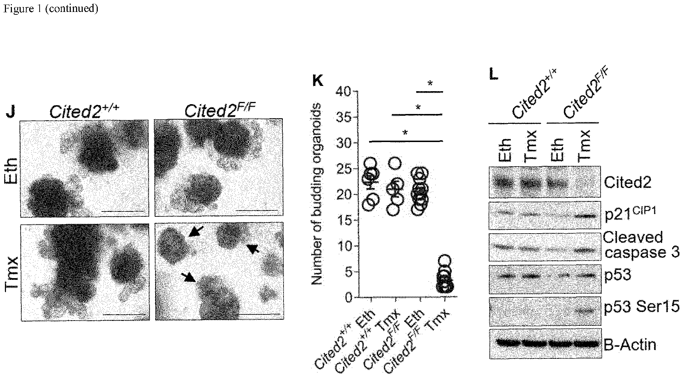

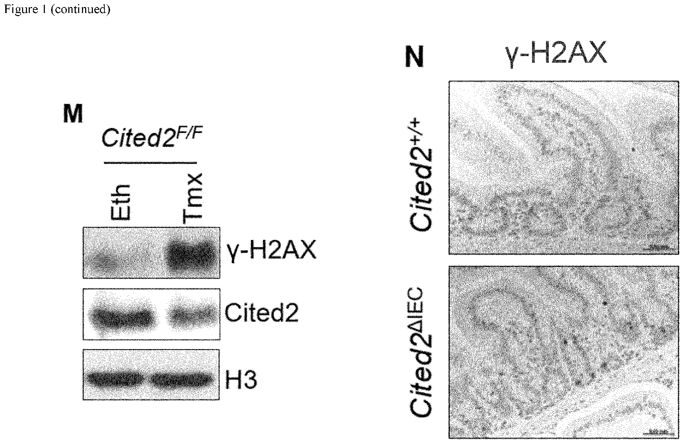

Figure 1 shows that CITED2 promotes survival of Lgr5⁺ ISCs in mice. (A) Detection of CITED2 expression in villus and crypt extracts of wild type mice by immunoblot analysis. n=3 mouse samples. (B) Immunohistochemistry (IHC) for CITED2 from a small intestine (SI) section of a wild type mouse. Bottom image shows higher magnification of the dashed box in the upper panel. (C) Histological H&E staining of SI of control (left) and *Cited2*-deficient mice (right). Black bars indicate crypt length. Note elongated crypt architectures (upper right panel) and abundant appearance of apoptotic bodies (arrows) in the SI four days after *Cited2* ablation. (D) Histological scoring evaluating crypt size of small intestines shown in (c). n=4 mice. Mean ± SD; t-test, *p<0.05. (E) IHC for activated caspase-3 shows positive cells (arrows) in SI crypt bottom cells four days after *Cited2* deletion. (F) Quantitation of apoptosis. n=4 mice. Mean ± SD; t-test, *p<0.05. (G) IHC staining of intestinal tissues for OLFM4 (black staining) from control and experimental mice four days after Tmx induction. (H) Lgr5 mRNA expression (black staining) in small intestines (upper panel) and colons (lower panel) from indicated mice detected by RNA ISH (RNA scope; R.S.). Scale bar: 200 µm. (I) Immunofluorescence staining for GFP (light grey), which acts as surrogate marker for Lgr5. Arrows show small intestinal crypts with reduced Lgr5⁺ stem cells in *Cited2*-mutant mice 4 days after Tmx administration. DAPI marks cell nuclei (dark grey). (J) Light microscopy pictures of SI-derived organoids from indicated genotypes were established and 4-OH tamoxifen induced overnight for recombination. Images were taken 4 days after treatments. Arrows show growth-deficient organoids. Scale bar: 50 µm. (K) Scoring of organoid growth ability based on the number of budding organoids. Each data point (=n) represents average number of budding organoids, which was calculated from three-independent 24-well plates for every mouse. Mean ± SD; *p<0.05 by 1-way ANOVA with Bonferroni's multiple comparison test. (L and M) Western blot of protein lysates isolated from *Cited2*^{*+*/*+*} and *Cited2*^{*F*/*F*} intestinal organoids for the indicated markers. (N) IHC staining of small intestinal tissues for γ-H2AX (dark grey staining) from wild type and *Cited2*-deficient mice four days after Tmx induction. Hematoxylin (grey) was used as counterstaining.

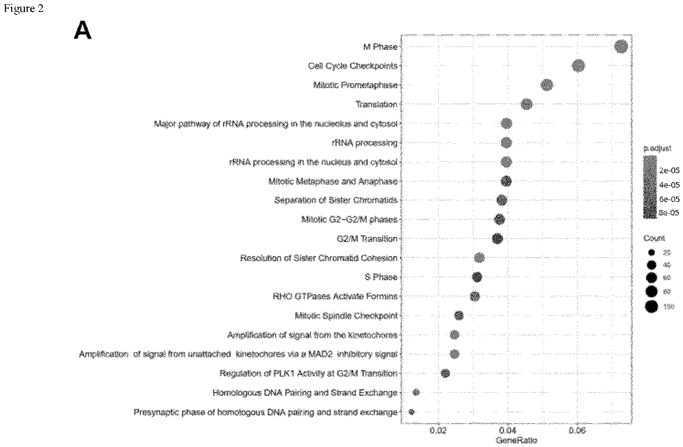

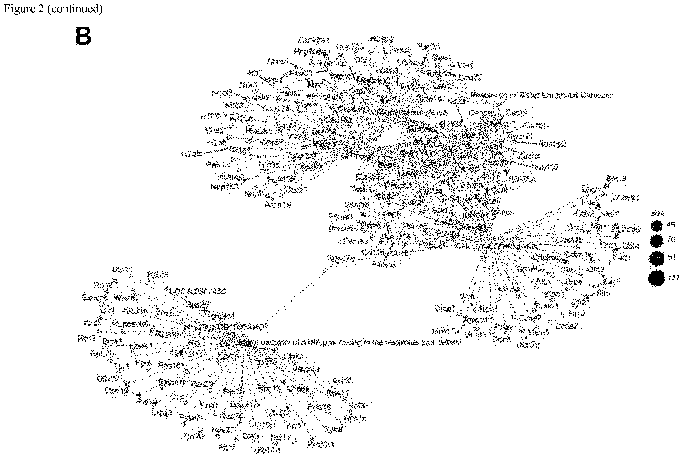

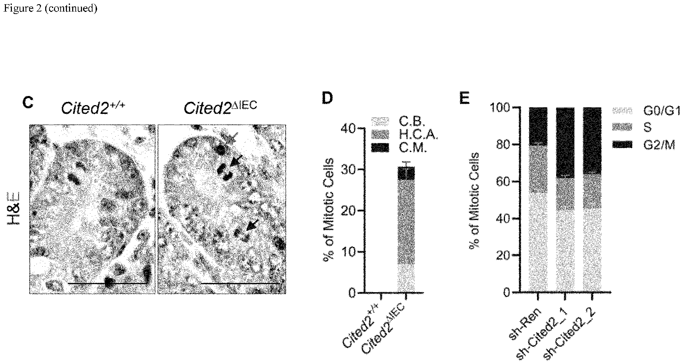

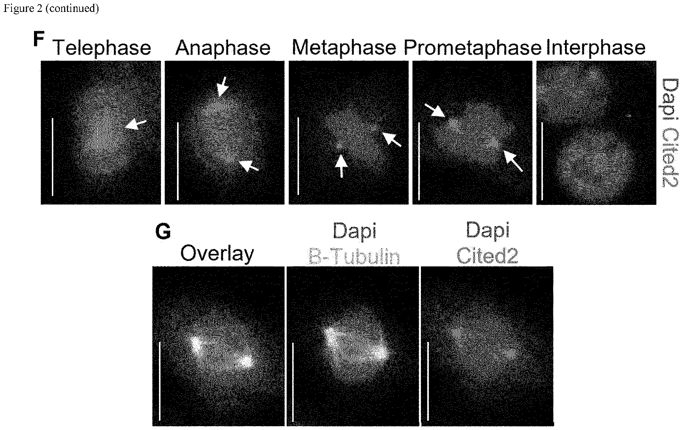

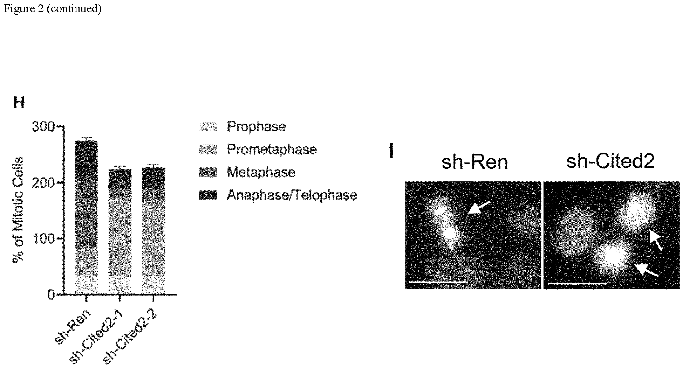

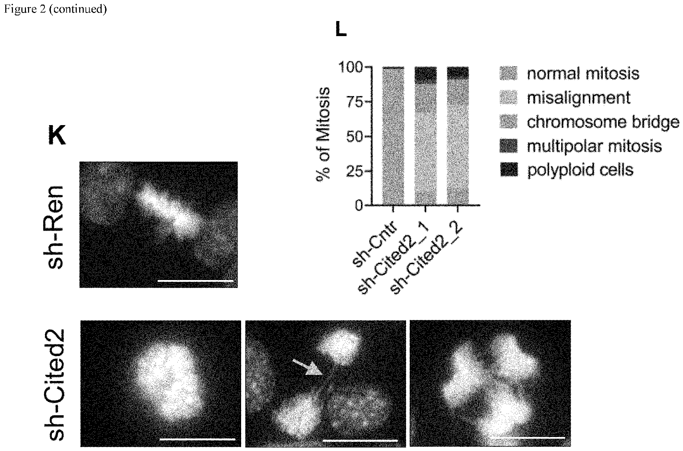

Figure 2 shows that CITED2 promotes faithful mitosis in intestinal epithelial cells. (A and B) Pathway analysis of the differentially expressed genes from SI tissue of *Cited2^{ΔIEC}* vs *Cited2*^{*+*/*+*} mice 18 hours after tamoxifen administration using "ReactomePA" R package. "enrichPathway" function was used to determine the top 20 significant enriched terms using "dotplot" function (A). In order to visualise which genes are involved with the top enriched terms, gene-concept network was plotted using "cnetplot" function (B). (C) Representative H&E staining pictures of SI tissues from control and *Cited2^{ΔIEC}* mice that were treated with tamoxifen for 18 hours. Black arrows indicate crypt epithelial cells with chromatin bridge mitotic errors. The grey arrow shows a crypt intestinal epithelial cell with hypercondensed chromatin defect. Scale bar: 50 µm. (D) Scoring of mitotic errors from H&E-stained intestinal tissue sections for the indicated genotypes. C.B. (chromatin bridge), C.M. (chromosome misalignment), H.C.A. (hypercondensed chromatin aggregates). Scale bar: 50 µm, n=3. (E) Analysis of the cell cycle in Mode-K cells 48 hours post CITED2 RNAi. Pie chart shows the proportion of cells in each phase. (F) Immunofluorescence staining of Mode-K cells during interphase or mitosis for CITED2. Arrows point-out dynamic CITED2 localization in different stages of mitosis. (G) Representative dual immunofluorescence staining of a mitotic Mode-K cell for the indicated markers. Scale bar: 10 µm. (H) Mitotic phase distribution following CITED2 RNAi. Approximately 500 cells were counted and scored for each condition. Mean ± SD. (I) Microscopic images of control and CITED2 knockdown Mode-K mitotic cells (arrows). Note that sh-CITED2 mitotic cells display prometaphase-like morphologies, which lack metaphase plate chromosome alignment. Scale bar: 10 µm. (J) Representative immunofluorescence staining of a mitotic Mode-K cell for kinetochore marker Bub1 shows dispersed chromosome arrangement following CITED2 silencing. Scale bar: 10 µm. (K) Microscopic images show aberrant mitosis in CITED2 RNAi Mode-K cells. CITED2-deficient cells display frequently chromosome misalignment (down panel, left), chromosome mis-segregation (down panel, middle), and multipolar mitosis (down panel, right) compare to control cells, which typically shows proper metaphase chromosome alignment (upper panel). Light grey arrow shows chromosome bridge, dark grey arrow shows a lagging chromosome during cell division. Scale bar: 10 µm. (L) Scoring of mitotic errors shown in (K). At least 500 cells were counted and scored for each condition.

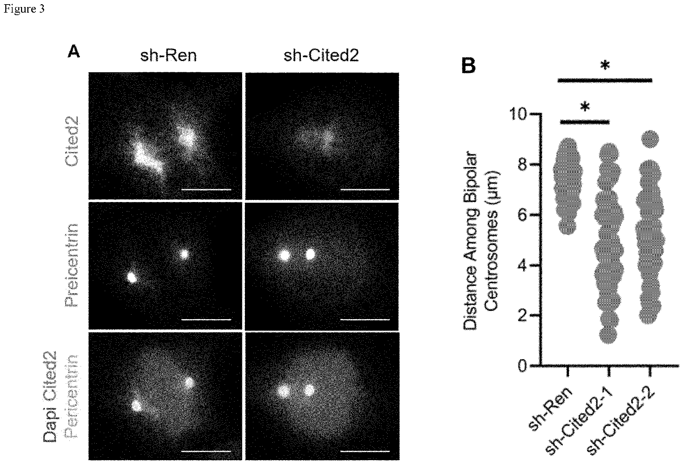

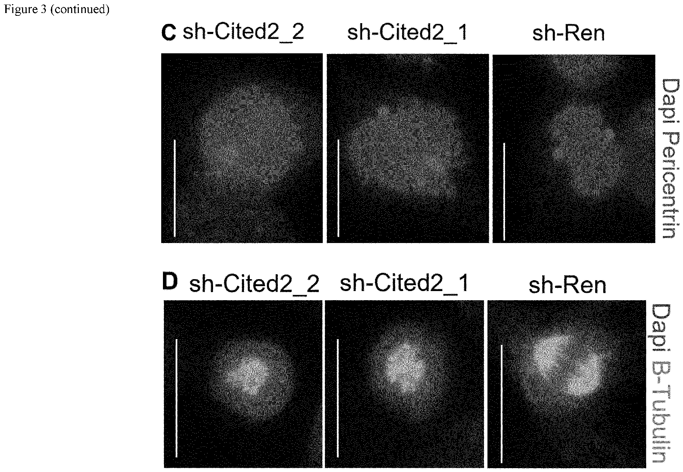

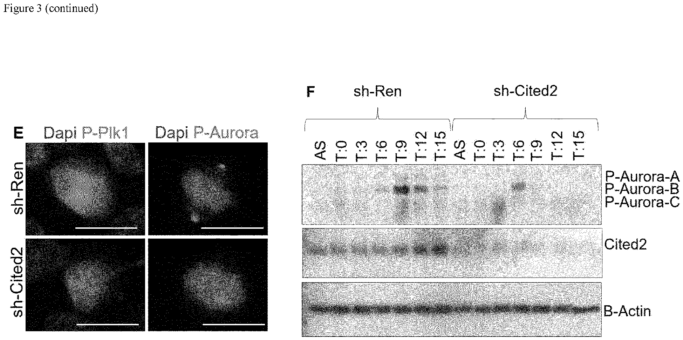

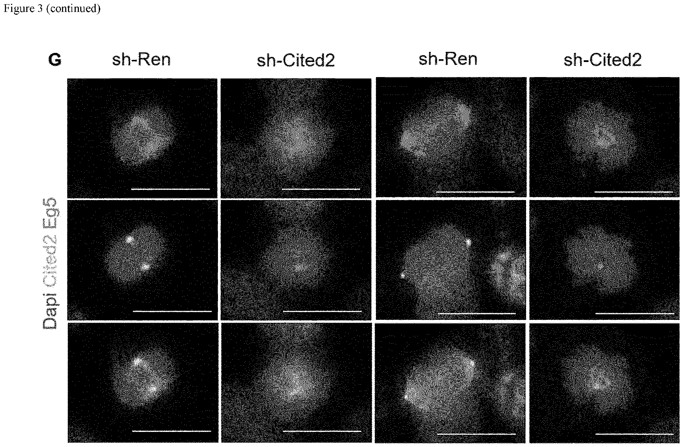

Figure 3 shows that CITED2 is essential for proper centrosome segregation and subsequent bipolar spindle assembly in Mode-K cells (A) Immunofluorescence staining showing distinct CITED2 localization at centrosomes (marked by Pericentrin antibody) during mitosis. Scale bar: 10 µm. (B) Scoring of inter-centrosome distances in both sh-Ren and sh-CITED2 Mode-K cells. Mean ± SD; *p<0.05 by 1-way ANOVA with Bonferroni's multiple comparison test. At least 100 centrosomes were counted for each condition. Note lack of bipolar centrosome separation in CITED2 RNAi cells. (C) Immunofluorescence staining for Pericentrin (light grey) shows supernumerary centrosomes in CITED2-deficient mitotic cells. Scale bar: 10 µm. (D) Representative immunofluorescence staining for B-tubulin (light grey) shows formation of chaotic monopolar mitotic spindles in CITED2 RNAi cells. Scale bar: 10 µm. (E) Representative immunofluorescence staining of mitotic Mode-K cells either for phospho Plk1 or phospho Aurora kinases. Scale bar: 10 µm. (F) Western blotting analysis of control and CITED2 knock-down cells for the indicated markers. Cells were synchronized by double-thymidine block and then were released for the indicated times. (G) Dual Immunofluorescence staining of mitotic cells for Kinesin-5 Eg5 (red) and CITED2 (green) show monopolar diminished Eg5 localization following CITED2 silencing. Scale bar: 10 µm.

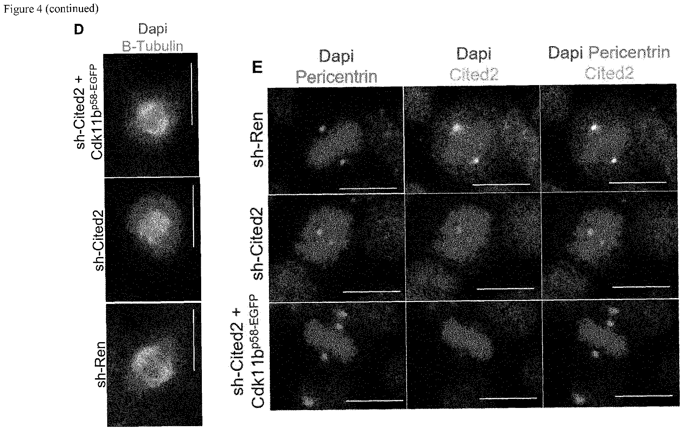

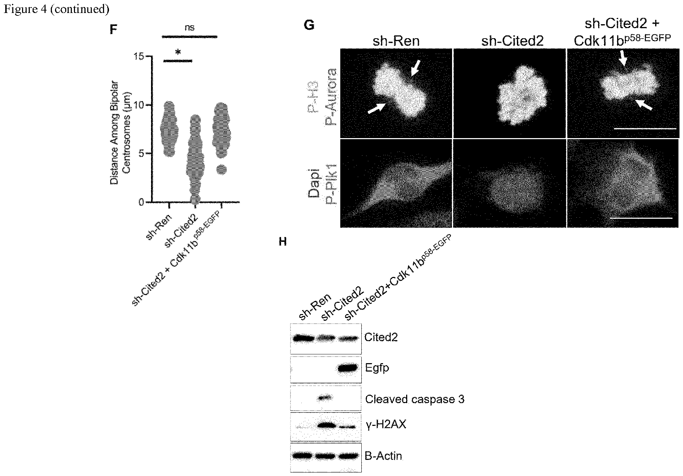

Figure 4 shows that Cdk11b rescues mitotic catastrophe in CITED2-deficient Mode-K cells. (A) Scheme of the experimental workflow of SILAC (stable isotype labelling of amino acids in culture) labelling of Mode-K cells during interphase and mitosis. Cells were incubated with SILAC medium containing light or heavy version of lysine and arginine, until the isotype-labeled amino acids have been full incorporated into proteomes (> 5 passages). After labeling, cells were synchronized with double thymidine (DT) block and then released into cell cycle. Samples representing G1/S (interphase; 0 hours post DT) and G2/M (mitosis; 9 hours post DT) were taken for the subsequent mass spectrometry analysis. Cell lysates from each condition were precipitated either with CITED2 (C) or isotype control antibodies (IgG). (B) Representative confocal immunofluorescence staining of a mitotic Mode-K cell for Cdk11b (red) and CITED2 (green) shows colocalization of two proteins. Scale bar: 10 µm. (C) Immunoblot analyses of immunoprecipitations of HA-tagged CITED2 or IgG control antibody. Cdk11b^{p58-EGFP} over-expressing Mode-K cells (upon 24 hours DOX treatment), were transiently transfected with a plasmid encoding HA-tagged murine CITED2. Cell lysates were then immunoprecipitated either with HA-tag or IgG antibodies. Western blotting was performed with the indicated antibodies. (D) Representative immunofluorescence staining of mitotic Mode-K cells from indicated groups for the B-tubulin (light grey). Scale bar: 10 µm. (E) Dual immunofluorescence staining of mitotic Mode-K cells for centrosome marker pericentrin (red) and CITED2 (green) shows bipolar centrosome separation in CITED2-deficient cells upon forced Cdk11b^{p58-EGFP} expression. Scale bar: 10 µm. (F) Scoring of inter-centrosome distances in the indicated groups. Mean ± SD; *p<0.05 by 1-way ANOVA with Bonferroni's multiple comparison test. At least 100 centrosomes were counted for each condition. ns: not significant. (G) Representative immunofluorescence staining of mitotic Mode-K cells for the indicated proteins. White arrows show phospho Aurora staining at the spindle poles of control and Cdk11b^{p58-EGFP}-expressing CITED2 knock-down cells. Scale bar: 10 µm. (H) Immunoblotting of CITED2-proficient and CITED2-deficient cell lysates for the indicated markers.
Figure 5 shows that CITED2 mediates colorectal cancer maintenance by controlling cell cycle progression and counteracting anti-tumor immunity. (A) Propidium iodide (PI) staining of human SW48 CRC cells 48 hours after 50nM control or CITED2 antisense gapmer treatment (upper panel). Dual immunofluorescence staining of SW48 cells for CITED2 (green) and B-tubulin (red) from (a). White arrows show mitotic cells. Note collapsed bipolar spindle formation in SW48 cells following CITED2 inhibition. Scale bar: 10 µm. (B) qRT-PCR of CITED2 expression in SW48 cells 48 hours after infection with control or CITED2-specific gapmers. Mean ± SD; t-test, *p<0.05. (C) Representative endoscopy images of colonic tumors in AOM-DSS-treated mice from indicated genotypes. Tmx treatment started after tumor establishment. Light grey dashed line marks tumor remnant. n=3 mice. (D) H&E staining pictures of colon tissues from indicated mice 2-week after tamoxifen administration. Colonic tumors are marked with black dashed lines. (E and F) Quantification of colonic tumor volume and number in mice from (D). Mean ± SD; t-test, *p<0.05. n=3 mice. (G) Growth rate of subcutaneous APT tumors in response to treatments as indicated. D: doxycycline. T: treatment; either isotype (Iso) or Anti-PD-1. Mice were treated every 3 days. n= 5 mice for each group. Mean ± SD; *p<0.05 by 2-way ANOVA with Bonferroni's multiple comparison test. (H) Representative photos of subcutaneous APT tumors at day 30. (I) Representative H&E and IHC pictures of colonic tumors from (H) for the indicated markers. (J) Growth rate of subcutaneous APT tumors in immunodeficient NSG mice. D: doxycycline. Mice were treated with doxycycline every 3 days. n= 6 mice for each group. Mean ± SD; *p<0.05 by 2-way ANOVA with Bonferroni's multiple comparison test. (K) Representative photos of subcutaneous APT tumors in NSG mice at day 27. (L) Representative H&E and Ki67 IHC pictures of colonic tumors from (K). (M) Immunofluorescence staining of subcutaneous APT tumors from both immunoproficient (BL6) and immunodeficient (NSG) mice for EpCAM (red). DAPI (blue) stains nuclei. Scale bar: 500 µm.

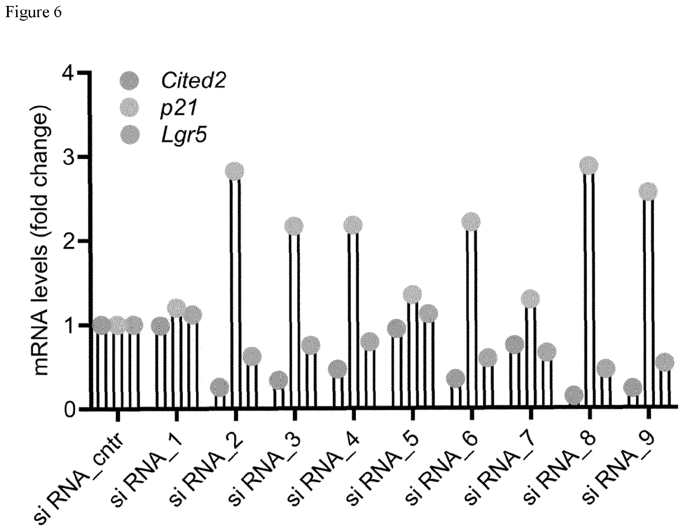

Figure 6 shows that knockdown of CITED2 reduces Lgr5 expression and induces p21 upregulation. The bars with dots each represent (from left to right) CITED2, p21, and LrpS, respectively.
SEQ ID No. 1: gctgttgactcgatcgaaacc shows the human CITED2-specific hairpin HSH000441-32-LVRU6MP.
SEQ ID No. 2: gcagatattactgcacaaact shows the human CITED2-specific hairpin HSH000441-34-LVRU6MP.
SEQ ID No. 3: gcttcgcgccgtagtctta shows a non-specific hairpin encoded in a control vector, CSHCTR001-1-LVRU6MP.
SEQ ID No. 4: 5'-TGCCGCCCAATGTCATAGACAC-3' shows qRT-PCR *mCited2* forward primer.
SEQ ID No. 5: 5'-AGAGTTCGGGCAGCTCCTTGAT-3' shows qRT-PCR *mCited2* reverse primer.
SEQ ID No. 6: 5'-GAGTCAACCCAAGCCTTAGTATCC-3' shows qRT-PCR *mLgr5* forward primer.
SEQ ID No. 7: 5'-CATGGGACAAATGCAACTGAAG-3' shows qRT-PCR *mLgr5* reverse primer.
SEQ ID No. 8: 5'-GCCACTTTCCAATTTCAC-3' shows qRT-PCR *mOlfm4* forward primer.
SEQ ID No. 9: 5'-GAGCCTCTTCTCATACAC-3' shows qRT-PCR *mOlfm4* reverse primer.
SEQ ID No. 10: 5'-TTGGATCCTACACCGTGACC-3' shows qRT-PCR *mCdk11b* forward primer.
SEQ ID No. 11: 5'-TAGTTCTGGGGCACGATACC-3' shows qRT-PCR *mCdk11b* reverse primer.
SEQ ID No. 12: 5'-GCTACCTGGGTCAGGACTTG-3' shows qRT-PCR *mIrf1* forward primer.
SEQ ID No. 13: 5'-CAGAGAGACTGCTGCTGACG-3' shows qRT-PCR *mIrf1* reverse primer.
SEQ ID No. 14: 5'-AGATCCCCTGGATGCATGC-3' shows qRT-PCR *mIrf2* forward primer.
SEQ ID No. 15: 5'-CAGTTTCTGAAGAGCGGAGCA-3' shows qRT-PCR *mIrf2* reverse primer.
SEQ ID No. 16: 5'-ATGTACCGAGACTACGGGGAA-3' shows qRT-PCR *mIrf3* forward primer.
SEQ ID No. 17: 5'-CTGCTGCTGTCGATGCTTG-3' shows qRT-PCR *mIrf3* reverse primer.
SEQ ID No. 18: 5'-ACAGGGCGTTTTATCTTGCG-3' shows qRT-PCR *mIrf7* forward primer.
SEQ ID No. 19: 5'-TCCAAGCTCCCGGCTAAGT -3' shows qRT-PCR *mIrf7* reverse primer.
SEQ ID No. 20: 5'-ATGTCAGCTCCCCAAATGTCC-3' shows qRT-PCR *mMxl* forward primer.
SEQ ID No. 21: 5'-TGCCTACAGCCACCCTGG-3' shows qRT-PCR *mMxl* reverse primer.
SEQ ID No. 22: 5'-AGTGAGGAGCTGCAGAAGTACG-3' shows qRT-PCR *mMx2* forward primer.
SEQ ID No. 23: 5'-ACTTGGTAGTTCTGTGGAGGTT-3' shows qRT-PCR *mMx2* reverse primer.
SEQ ID No. 24: 5'-TTACTGCCACGGCACAGTCA-3' shows qRT-PCR *mInf-g* forward primer.
SEQ ID No. 25: 5'-AGTTCCTCCAGATATCCAAGAAGAGA-3' shows qRT-PCR *mlnf⁻g* reverse primer.
SEQ ID No. 26: 5'-AGCTCCAAGAAAGGACGAACAT-3' shows qRT-PCR*MInfb1* forward primer.
SEQ ID No. 27: 5'-GCCCTGTAGGTGAGGTTGATCT-3' shows qRT-PCR *mInfb1* reverse primer.
SEQ ID No. 28: 5'-TGGCTAGGCTCTGTGCTTTC-3' shows qRT-PCR *MInfa4* forward primer.
SEQ ID No. 29: 5'-CTTCAGGCAGGAAAGAGGGG-3' shows qRT-PCR *mInfa4* reverse primer.
SEQ ID No. 30: 5'-CACGGTCGCTGTAGAAGTAAAG-3' shows qRT-PCR *mInfaR1* forward primer.
SEQ ID No. 31: 5'-TCTCCTCCTCTTCGTTGGAATA-3' shows qRT-PCR *mInfaR1* reverse primer.
SEQ ID No. 32: 5'- CTTGAACCCTGTCGTATGCTGG -3' shows qRT-PCR *mInfgR1* forward primer.
SEQ ID No. 33: 5'-TTGGTGCAGGAATCAGTCCAGG-3' shows qRT-PCR *mInfgR1* reverse primer.
SEQ ID No. 34: 5'-GAATCCGGAATCTAAGACCATCAA-3' shows qRT-PCR *mCxCl10* forward primer.
SEQ ID No. 35: 5'-GTGCGTGGCTTCACTCCAGT-3' shows qRT-PCR *mCxCl10* reverse primer.
SEQ ID No. 36: 5'-TGCGGACTACAAGCGAATCACG-3' shows qRT-PCR *mPD-L1* forward primer.
SEQ ID No. 37: 5'-CTCAGCTTCTGGATAACCCTCG-3' shows qRT-PCR *mPD-L1* reverse primer.
SEQ ID No. 38: 5'-GCCTCTCATTGTCACCGAAGAAC-3' shows qRT-PCR *mStat1* forward primer.
SEQ ID No. 39: 5'-TGGCTGACGTTGGAGATCACCA -3' shows qRT-PCR *mStat1* reverse primer.
SEQ ID No. 40: 5'-GAACCAACTCTCCATTGCCTGG-3' shows qRT-PCR *mStat2* forward primer.
SEQ ID No. 41: 5'-CGTAAGAGGAGAACTGCCAGCT-3' shows qRT-PCR *mStat2* reverse primer.
SEQ ID No. 42: 5'-ACCCTTTCCAGTCTGGGTCT-3' shows qRT-PCR *mIgs-15* forward primer.
SEQ ID No. 43: 5'-TCGCTGCAGTTCTGTACCAC-3' shows qRT-PCR *mIgs-15* reverse primer.
SEQ ID No. 44: 5'- GCTGGTGAAGCAGAGAGACTCAG-3' shows qRT-PCR *mMHC-I* forward primer.
SEQ ID No. 45: 5'- GGTGACTTTATCTTCAGGTCTGCT-3' shows qRT-PCR *MHC-I* reverse primer.
SEQ ID No. 46: 5'-GTGACGTTGACATCCGTAAAG-3' shows qRT-PCR *mB-actin* forward primer.
SEQ ID No. 47: 5'-GCCGGACTCATCGTACTCC-3' shows qRT-PCR *mB-actin* reverse primer.
SEQ ID No. 48: 5'-TGCCGCCCAATGTCATAGACAC-3' shows qRT-PCR *hCited2* forward primer.
SEQ ID No. 49: 5'-CAGCTCCTTGATGCGGTCCAAA-3' shows qRT-PCR *hCited2* reverse primer.
SEQ ID No. 50: 5'-CAAGGCCAACCGCGAGAAGATGAC-3' shows qRT-PCR *hB-actin* forward primer.
SEQ ID No. 51: 5'-GCCAGAGGCGTACAGGGATAGCACA-3' shows qRT-PCR *hB-actin* reverse primer.

The following table shows siRNA sequences according to the present invention that target CITED2. In bold are siRNA sequences that very efficiently target CITED2. siRNA_7 (underlined) shows intermediate efficiency.

| **si RNA No** | **Q-RT-PCR Sample Info** | SEQ ID No. | **target sequence** |
|---|---|---|---|
| | | | |
| J-015358-05, Cited2 | si RNA_1 | 52 | GAAAUGGGUUUGGACCGC A |
| J-015358-06, Cited2 | si RNA_2 | 53 | GCUGUUGACUCGAUCGA AA |
| J-015358-07, Cited2 | si RNA_3 | 54 | GUUCUUAUGUCCUUGGU GA |
| J-015358-08, Cited2 | si RNA_4 | 55 | AACCAGCACUUCCGAGA UU |
| s223110, Cited2 | si RNA_5 | 56 | UAUGACAUUGGGCGGCAG C |
| s20283, Cited2 | si RNA_6 | 57 | AAGGACAUAAGAACUUC CU |
| s223109, Cited2 | si RNA_7 | 58 | UGAUGGUUGAAAUACUGG U |
| s20281, Cited2 | si RNA_8 | 59 | AUUUCUAUCACCAAGGA C |
| s20282, Cited2 | si RNA_9 | 60 | UCGUCGAUGAAAUCAGU GU |
| | | | |

### Examples

### Materials and Methods

### Ethics statement

All experiments were performed under the UK Home Office guidelines (Licence 60/4183) and by adhering to the ARRIVE guidelines with approval from the local ethical review committee of the University of Glasgow as well as reviewed and approved by the Regierungspräsidium Darmstadt, Darmstadt, Germany.

### Mice model

Male and female mice at 6 to 12 weeks of age were used for experiments. All mice were housed in specific pathogen-free facilities at the Beatson Institute, Glasgow and at the Georg-Speyer-Haus, Frankfurt am Main. Mice were kept in individually ventilated conventional cages in an animal room at constant temperature (19-23 °C) and humidity (55% ± 10%) under a 12-h light-dark cycle and were allowed access to standard diet and water ad libitum. Animal work was performed in the mixed C57BL/6/FVB background. The alleles used for this study were as follows: *Cited2*^{*F*/*F*} (21), *villin-creERT2* (22), *Lgr5-creERT2* (1) *Lgr5-GFP-DTR* (19), *Rosa-creERT2* (45). Recombination by *villin-creERT2* was induced either with one intraperitoneal injection (IP) of 80 mg kg⁻¹ tamoxifen on day 0 and day 1 or by daily oral administrations of 1 mg tamoxifen dissolved in ethanol/oil mixture. Analysis of *villin-creERT2-*induced mice were at indicated days after induction. Cohort mice were aged until they showed clinical signs of intestinal tumorigenesis, indicated by anemia, hunching and/or weight loss.

Experimental colitis was induced by administrating 3% dextran sodium sulphate (DSS) (36-50 kDa, MP Biomedicals, CA) in drinking water for 6 days, followed by six additional days of regular drinking water. Histology scores were scored as previously described (46). Colitis-associated carcinogenesis (CAC) was performed as previously described using 10 mg/kg Azoxymethane (AOM) (Sigma-Aldrich, St. Louis, MO) and 2% DSS (molecular weight, 36-50 kDA; MP Biomedicals, #SKU 0216011090) (47).

Endoscopy was performed at around day 70 before and after tamoxifen treatment on anesthetized mice using a mouse mini endoscopy system (Karl Storz). Recorded videos from the endoscopy system were opened and viewed in VLC Media Player and still images were captured from these videos. For subcutaneous tumour experiment, APT organoids were mechanistically disrupted, incubated with Accutase to generate a single cell suspension and reconstituted in PBS 20% Matrigel (Corning) before injection. Tumor sizes were measured at the indicated time after transplantation and tumor volume quantified as ½ (width² x length). 3 days after tumor detection, mice were given doxycycline (Dox) every three days throughout the experiment. To study combination efficacy with immunotherapy, mice were injected i.p. with 100µl nVivoMAb anti-mouse PD-1(Hoelzel; BE0146) and InVivoMab mouse IgG2b isotype control (Hoelzel; BE0086) every 3 days, following Dox treatment. Both male and female C57BL/6J mice were ordered from Charles River and Janvier. Mice were randomly distributed in subcutaneous transplantation experiments.

### Histological analysis

Mouse intestine, colon and tumor samples were fixed overnight in 10% formalin, washed with PBS and 70% ethanol, and embedded in paraffin. The paraffin blocks were cut into 4-6 µm sections using a microtome. Sections were then deparaffinized, rehydrated and stained with hematoxylin and eosin. Immunohistochemistry was performed either manually using standard techniques or Bond-Max (Leica) and the Bond Polymer Refine Detection System (Leica) as described by the manufacturer's instructions using the following antibodies provided also in the key resource table, CITED2 (1/20; Santa Cruz sc-21795), Olfm4 (1/250; Cell Signaling 14369), Cleaved Caspase-3 (1/500; Cell Signaling 9579), lysozyme (1/200; Dako A099), GFP (1/1000; Abcam ab6556), γ-H2AX (1/500; Upstate 05636), γ-H2AX (1/1000; Cell Signaling 9718S), RFP (1/200; Rockland 600-401-379), Ki-67 (1/200; Leica Biosystems, number NCL-L-Ki67-MM1), E-cadherin (1/500; BD Biosciences 610182), CD3 (Ready-to-use; Dako, IR503), CD4 (1/100; Cell Signaling 25229), CD8 (1/500; Cell Signaling 98941), PD-L1 (1/200; R&D AF1021). For each antibody, staining was performed on at least three mice of each genotype, with representative images shown for each staining. Apoptosis was scored from caspase-3-stained sections. Proliferation levels were assessed by immunostaining using antibodies against Ki67. Crypt numbers and sizes were calculated from H&E-stained intestinal sections. For each analysis, at least 100 full crypts were scored from at least three mice of each genotype using an Aperio ScanScope CS2 (Leica), and positive cells were counted using Aperio Image Scope. Apoptosis index represents percentage of average number of cells that were calculated by dividing number of cleaved caspase 3+ cells to the total number of crypt cells per crypt for each mouse. For microscopy, we employed the Zeiss Axio Vision microscope. Images were acquired using AxioVision software provided by Zeiss. Negative controls included the omission of the primary antibody. Immunofluorescent staining was performed manually. Briefly, cryostat sections were blocked with 0.3 % hydrogen peroxide and subsequently 3% donkey serum in PBS and incubated with primary antibody in blocking buffer overnight at 4°C. After washing with PBS three times, slides were incubated with appropriate fluorescent secondary antibodies for 1 hour at room temperature and covered with ProLong Gold antifade mounting medium with DAPI (Thermo Fisher Scientific; P36931). Images were taken with an Axio Imager 2 (Zeiss). Tumor volume was determined by measuring the area of each tumor in serial sections using AxioVision software.

### Intestinal villus, crypt, and epithelial cell extraction

10 cm portions of mouse small intestine were flushed with PBS and opened longitudinally. Villi were removed with a glass coverslip. A small piece of intestines was incubated with 2 mM EDTA/PBS for 30 min at 4 °C followed by 5 min of vigorous shaking for a total of 5 incubations. The last two fraction contained intestinal crypts and were used for downstream analysis. Intestinal epithelial cell isolation was carried out as previously described (48).

### Immunoblotting

Protein lysates were homogenized using the Precellys 24 (Stretton Scientific) in RIPA buffer with complete protease inhibitors (Roche) and, protein concentrations were determined using a BCA Protein Assay Kit (Pierce). Proteins were resolved via 10-12% SDS-PAGE and transferred to nitrocellulose membranes. After blocking the membranes in TBS containing 5% BSA (Sigma), 0.02% Triton X-100 for 1 h, primary antibodies were added in block solution at the following dilutions: CITED2 (1/500; Insight Biotechnology sc-21795), CITED2 (1/500; R&D Systems AF5005), Cytokeratin 20 (1/500; Santa Cruz sc-271183), Villin (1:1000; Santa Cruz sc-7672), Olfm4 (1/250; Cell Signaling 14369), Histone H3 (1/5000; abcam ab1791), Cleaved Caspase-3 (1/1000; NEB 9661), p21 (1/500; Insight Biotechnology sc-397), p53 (1/500; Insight Biotechnology sc-393031), phospho-p53 (Ser15) (1/1000; NEB 9284), phospho-Aurora A (Thr288)/Aurora B (Thr232)/Aurora C (Thr198) (1/1000; Cell Signaling 2914S), GFP (1/500; Proteintech 50430-2-AP), γ-H2AX (1/1000; Cell Signaling 9718S), Phospho-Histone H3 (Ser10) (1/1000; Cell Signaling 9706S), PITSLRE/Cdk11 (1/500; Santa Cruz sc-377296), HA-tag (1/200; ThermoFisher 26183), and β-actin (1/5000; Sigma A2228). Primary antibody incubations were carried out at 4 °C overnight. After washing, the appropriate HRP-conjugated secondary goat antibodies (Dako) were added diluted at 1/10,000 in block for 1 h. Antibody binding was detected using ECL Western Blotting Substrate (Pierce).

### Crypt and cell culture experiments

Human SW48 CRC cells and murine small intestine Mode-K cells were cultured and maintained in a commercial DMEM medium with 10% FBS in a culture incubator with 5% CO₂ and 37°C temperature. Mouse small intestines were opened longitudinally and washed with PBS. Crypts were isolated as previously described (49). Isolated crypts were seeded in growth factor-reduced Matrigel (BD Bioscence), plated in 24-well plates in Advanced DMEM/F12 (ADF, Life Technologies) supplemented with 1% glutamine, 1% penicillin/streptomycin, 10 mM HEPES (Life Technologies), EGF (50 ng ml⁻¹, Peprotech), Noggin (100 ng ml⁻¹, Peprotech) and mR-spondin (500 ng ml⁻¹; R&D Systems). Growth factors were added every other day. 4-hydroxytamoxifen (500 nM; Sigma, H7904) was used overnight to induce Cre in culture. Organoid number and bud protrusions were counted on microscopic images using an Evos microscope (Thermo Fisher Scientific; 20X objective). Allele-specific primers for PCR genotyping are available on request. For confocal immunofluorescence microscopy, organoids were collected and fixed with 4% PFA in PBS. Organoids were then permeabilized with cold 0.2% Triton X-2100 in PBS and blocked with 3% BSA and 0.1% Tween-20 in PBS. After blocking, the organoids were incubated overnight with primary antibody (prepared in 1% BSA in PBS), washed three times, and then incubated with secondary antibody for 1 h. After washing, the organoids were transferred onto microscope slides and mounted with mounting medium (Vector Laboratories) under a coverslip with DAPI to visualize nuclei. The primary antibodies used were CITED2 (1/15; Insight Biotechnology sc-21795), lysozyme (1/1000; Dako A099), GFP (1/200; Abcam ab6556). The secondary antibodies used were Donkey anti-Mouse Alexa 647, Donkey anti-Goat Alexa 488 and Donkey anti-rabbit Alexa 546 (all were obtained from Life Technologies). Samples were examined using an Olympus FV1000 inverted laser scanning confocal microscope. SW48 human CRC cell line and mouse intestinal Mode-K epithelial cells were cultured in RPMI medium supplemented with 10% FBS, 1% Glutamine and 1% penicillin/streptomycin. Lentiviral plasmids encoding reverse tetracycline transactivator (rtTA) and DOX-inducible EGFP-fused Cdk11b p58 isoform (Cdk11b^{p58-EGFP}) were purchased from VectorBuilder. Human embryonic kidney 293 (HEK293) cells were transfected with these vectors using Lipofectamine 2000 reagent according to manufacturer's instructions. Corresponding virus fractions were precipitated by ultra centrifugation at 20.000 Xg for 2 hours at 4°C and employed for infection of Mode-K cells. For the colony formation assay, growth medium was removed from cells and fix/stain solution containing 0.5 g Crystal Violet (0.05% w/v), 27 ml 37% Formaldehyde (1%), 100 mL 10X PBS (1X), 10 mL Methanol (1%) 863 dH20 to 1L was added to cover the dish for 20 minutes at room temperature. After removal of fix/stain solution, dishes were washed by dipping into bucket of water in the sink with the water continuing to run. After air drying of dishes, colonies were counted by eye. For assessment of cell viability, 50 µg/mL propidium iodide was added to cell culture medium for 30 minutes at 37 °C and red fluorescence color was observed under the microscope. To assess microtubule regrowth potential of control and CITED2 RNAi Mode-K cells, the microtubules were depolymerized by placing the cells on ice for 30 min. These cells were then incubated with warm (37°C) medium for 1, 2 or 3 min and fixed with -20°C methanol and processed for immunofluorescence microscopy. Mode-K cells were synchronized with a double-thymidine block method. Briefly, cells were blocked with 2 mM thymidine (Sigma, T1895) for 16 h, washed twice with PBS, released into fresh medium for 10 h and blocked again with 2 mM thymidine for 16 h. Finally, cells were released into fresh medium. G1/S and G2/M cells were collected at the indicated time points after the release from the second thymidine block. Progression into mitosis was verified by Western blotting using phospho-histone H3 antibody. To arrest cells at G2/M, nocodazole (absource S2775) was added to culture medium at 25 ng/mL for 6 hours. To determine percentage of cells at different cell cycle stages, Propidium Iodide Flow Cytometry Kit (abcam ab139418) was used according to manufacturer's instructions. Briefly, 500.000 cells were trypsinized, washed twice with PBS, fixed with 66% ethanol on ice and stored at 4 °C. At the day of measurement, the cells were washed twice with PBS, resuspended in PBS buffer, treated with RNase A (200 µg per mL) and stained with propidium iodide (50 µg per mL) at 37 °C in the dark for 20-30 minutes. Cell cycle profiles (propidium iodide area versus count) were measured with Fortesa (BD) cytometers and analyzed with FlowJo software. For immunofluorescence staining, unchallenged or infected Mode-K cells were rinsed in PBS, fixed in 4% paraformaldehyde for 15 min at RT, permeabilized (PBS containing 0.1% Triton X-100) for 10 min, blocked (PBS containing 2% bovine serum albumin and 2% normal donkey serum) for 30 min, and incubated with the following antibodies at the indicated dilution: CITED2 (1/50; R&D Systems AF5005), β-tubulin (1:100; Sigma T4026), Bub1 (1:100; Abcam EPR18947), Pericentrin (1:500; Abcam 4448), phospho Plk1 (T210) (1:500, Abcam 4448), phospho-Aurora (1/1000; Cell Signaling 2914S), Kif11/Eg5 (1/200; ThermoFisher PA3-16834), γ-H2AX (1/1000: Cell Signaling 9718S), Phospho-Histone H3 (Ser10) (1/1000; Cell Signaling 9706S), PITSLRE/Cdk11 (1/500; Santa Cruz sc-377296), Cyclin B1 (1/200; Cell Signaling 4138S), Cleaved Caspase-3 (1/500; Cell Signaling 9579), phospho-Ser/Thr-Pro-MPM-2 (1/2000; Sigma 05-368). Secondary antibodies conjugated to Alexa Fluor 488, 555, and 647 (Molecular Probes) were used at 1:1000 dilution. After a 5-min wash in PBS containing 4',6-diamidino-2-phenylindole (DAPI), the coverslips were mounted in ProLong Diamond (Thermo Fisher) and analysed by fluorescent microscopy.

### RNAscope assay

*In situ* hybridization (ISH) analysis was performed using the RNAscope 2.5 LS (Brown, 322100) detection kit (Advanced Cell Diagnostics, Hayward, CA) on a Leica Bond Rx autostainer strictly according to the manufacturer's instructions. Staining was performed on 4 µm formalin fixed paraffin sections which were cut and then placed in a 60°C oven for 2 hours prior to staining. To ensure the quality and integrity of the available RNA the tissue being investigated was tested with the positive control probe. Only after probe quality control was ensured were the results evaluated. To further ensure accuracy and integrity of the staining a negative control probe (mm-DapB, 312038) was used to confirm that the tissue staining seen was accurate due to binding with the target probe and not non-specific.

Probes: Mm_*Lgr5* (312171), Mm_*Olfm4* (311831), positive control probe *Ppib* (313918) and negative control probe *DapB* (312038). All reagents and probes were purchased from Advanced Cell Diagnostics.

### RNA isolation and quantitative RT-PCR

Samples were homogenized using the Precellys 24 (Stretton Scientific) in RNA lysis buffer using a Qiagen RNeasy Mini Kit (Qiagen, Crawley, West Sussex, UK) according to the manufacturer's instructions. DNase I (Ambion/Applied Biosystems, Warrington, UK) was used to remove genomic DNA contamination from the RNA prior to RT-PCR, according to the manufacturer's instructions. One microgram of RNA was reverse transcribed to cDNA using Superscript II reverse transcription kit (Invitrogen). Briefly, RNA was incubated with 1 µl oligo dT (50 µM) and 1 µl dNTP Mix (10 mM each) in a total volume of 13 µl at 65°C for 5 min. The mixture was cooled on ice, and 4 µl of 5X First Strand Buffer, 1 µl of dithiothreitol (DTT; 0.1 M), 1 µl of RNase OUT (40 U/µl), and 1 µl of SuperScript II reverse transcription (200 U/µl) was added. Reverse transcription was performed at 42°C for 60 min. cDNA was diluted 1:5 with RNase-free water. qRT-PCR was performed with a 96-well StepOne real-time PCR machine (Applied Biosystems) using 2X FastStart Universal SYBR Green Master Mix (Roche). The reaction mixture without a template was run as a control. The reaction conditions were as follows: 95 °C for 15 min, followed by 40 cycles of three steps consisting of denaturation at 94 °C for 15 s, primer annealing at 60°C for 30 s, and primer extension at 72 °C for 30 s. A melting curve analysis was performed from 70 °C to 95 °C in 0.3 °C intervals. B-actin was used to normalize for differences in RNA input. Differential expression of mRNAs was calculated using the ΔΔCt method.

### Gene knockdown

CITED2 and the control shRNA encoding lentiviral psi-LVRU6MP plasmids were obtained from Genecoopeia. Human CRC cell lines were transduced either with human CITED2-specific hairpins, HSH000441-32-LVRU6MP (gctgttgactcgatcgaaacc; SEQ ID No. 1) and HSH000441-34-LVRU6MP (gcagatattactgcacaaact; SEQ ID No. 2) or with non-specific hairpin encoding control vector, CSHCTR001-1-LVRU6MP (gcttcgcgccgtagtctta; SEQ ID No. 3). For infection, human 293T cells were transfected with individual shRNA vectors together with helper plasmids using Lipofectamine 2000. 48 hours after transfection, medium containing virus were collected, sterile-filtered and precipitated via centrifuge at 15.000 rpm for 2 hours. Target cells were then spinoculated in a 24-well plate for 1 hour at 32°C at 600 g. After a 4-6 hours recovery at 37°C, the cells were centrifuged in a tube and cultured in growth medium until analysis. Doxycycline-inducible shRNAs against mouse CITED2 were cloned in the RT3GEPIR vector (T3G-GFP-(miR-E)-PGK-Puro-IRES-rtTA3) via XhoI and EcoRI restriction site cloning as published before (50). RT3GEPIR was a gift from Johannes Zuber (Addgene plasmid #111169; http://n2t.net/addgene:111169; RRID:Addgene_111169). shRNA sequences were taken from the publication (51). As control shRNA we used shRen.713 (against renilla luciferase) that was already present in the RT3GEPIR vector. For gapmers application, 50 nM negative control antisense gapmer (Qiagen, 339515/NMOD LG00000002-DDA) or human CITED2-specific antisense gapmer (Qiagen, 339511/NMOD LG00825080-DDA) were simply added to cell culture medium for 48 hours and analyzed for the gene knockdown.

### RNA sequencing and pathway analysis

To prepare the library for RNA sequencing, 500 ng of total RNA was used. The library was generated with TruSeq mRNA Sample Preparation v2 Kit (Illumina; RS-122-2002). Quantification of cDNA libraries was performed with the QuantiFluor ds DNA System (Promega), and fragment size was determined using the Fragment Analyzer (Advanced Bioanalytical). 50-bp single-end sequencing was performed on the Illumina HiSeq 4000 platform. Read counts were analyzed in the R/Bioconductor environment (version 3.2) using the DESeq2 package (version 1.8.1). Differentially regulated genes were filtered to a minimum of twofold change and false discovery rate-corrected P value < 0.05.

EnrichR gene expression data analysis was performed using web-based EnrichR tool as previously described (52, 53). Pathway analysis was performed using "ReactomePA" R package as described before (54). Briefly, the differentially expressed genes in the RNA seq. were used as input and filtered for P adjusted < 0.05 and -1 > log₂ fold ratio > 1. The list of genes was annotated and Entrez ids were retrieved using "org.Mm.eg.db" R package. "enrichPathway" function was then used to determine the top significant enriched terms and "dotplot" function was used to plot the top 20 enriched terms with pvalue < 0.05. In order to visualize which genes are involved with the top enriched terms, gene-concept network was plotted using "cnetplot" function.

### Mass spectrometry analysis

Mass spectrometric analysis of Co-IP experiments was conducted using gel-based bottom-up proteomics as described previously (55). Briefly, proteins were eluted in LDS sample buffer (NuPAGE, Thermo Fisher Scientific) and separated by polyacrylamide gel electrophoresis. After staining with Coomassie Brillant Blue, the whole gel lane was excised as 23 individual bands and proteins were digested in the gel matrix with Trypsin (Promega). The extracted peptides were analyzed by liquid chromatography and tandem mass spectrometry (LC-MS/MS) on an EASY-nLC 1000 HPLC system coupled to a Q-Exactive quadrupole-Orbitrap hybrid mass spectrometer via a nano electrospray ion source (all Thermo Fisher Scientific). For protein identification and quantitation, the MS raw data was processed with the MaxQuant software (56) and the mass spectra were searched against the Uniprot murine reference proteome (downloaded 07-02-2019) and an integrated database of common laboratory contaminants. SILAC-based quantitation was enabled by setting the multiplicity to double labeling and considering light (Arg+0/Lys+0) and heavy (Arg+10/Lys+8) conditions.

### Statistical analyses

Appropriate statistical tests were used for the analysis of all experiments. The data are represented as mean values ± SD from three independent experiments (n = 3) unless otherwise noted. Student's t-test, 1-way ANOVA or 2-way ANOVA with Bonferroni's multiple comparison tests were performed using GraphPad Prism 7 (La Jolla, CA). Values ≤ 0.05 were considered significant.

### qRT-PCR primers

qRT-PCR primers specific for mouse (m) and human (h) genomes were as follows.
*mCited2* forward, 5'-TGCCGCCCAATGTCATAGACAC-3' (SEQ ID No. 4); *mCited2* reverse, 5'-AGAGTTCGGGCAGCTCCTTGAT-3' (SEQ ID No. 5);
*mLgr5* forward, 5'-GAGTCAACCCAAGCCTTAGTATCC-3' (SEQ ID No. 6); *mLgr5* reverse, 5'-CATGGGACAAATGCAACTGAAG-3' (SEQ ID No. 7);
*mOlfm4* forward, 5'-GCCACTTTCCAATTTCAC-3' (SEQ ID No. 8); *mOlfm4* reverse, 5'-GAGCCTCTTCTCATACAC-3' (SEQ ID No. 9);
*mCdk11b* forward, 5'-TTGGATCCTACACCGTGACC-3' (SEQ ID No. 10); *mCdk11b* reverse, 5'-TAGTTCTGGGGCACGATACC-3' (SEQ ID No. 11);
*mIrf1* forward, 5'-GCTACCTGGGTCAGGACTTG-3' (SEQ ID No. 12); *mIrf1* reverse, 5'-CAGAGAGACTGCTGCTGACG-3' (SEQ ID No. 13);
*mIrf2* forward, 5'-AGATCCCCTGGATGCATGC-3' (SEQ ID No. 14); *mIrf2* reverse, 5'-CAGTTTCTGAAGAGCGGAGCA-3' (SEQ ID No. 15);
*mIrf3* forward, 5'-ATGTACCGAGACTACGGGGAA-3' (SEQ ID No. 16); *mIrf3* reverse, 5'-CTGCTGCTGTCGATGCTTG-3' (SEQ ID No. 17);
*mIrf7* forward, 5'-ACAGGGCGTTTTATCTTGCG-3' (SEQ ID No. 18); *mIrf7* reverse, 5'-TCCAAGCTCCCGGCTAAGT -3' (SEQ ID No. 19);
*mMxl* forward, 5'-ATGTCAGCTCCCCAAATGTCC-3' (SEQ ID No. 20); *mMxl* reverse, 5'-TGCCTACAGCCACCCTGG-3' (SEQ ID No. 21);
*mMx2* forward, 5'-AGTGAGGAGCTGCAGAAGTACG-3' (SEQ ID No. 22); *mMx2* reverse, 5'-ACTTGGTAGTTCTGTGGAGGTT-3' (SEQ ID No. 23);
*mInf-g* forward, 5'-TTACTGCCACGGCACAGTCA-3' (SEQ ID No. 24); *mInf-g* reverse, 5'-AGTTCCTCCAGATATCCAAGAAGAGA-3' (SEQ ID No. 25);
*MInfb1* forward, 5'-AGCTCCAAGAAAGGACGAACAT-3' (SEQ ID No. 26); *mInfb1* reverse, 5'-GCCCTGTAGGTGAGGTTGATCT-3' (SEQ ID No. 27);
*MInfa4* forward, 5'-TGGCTAGGCTCTGTGCTTTC-3' (SEQ ID No. 28); *mInfa4* reverse, 5'-CTTCAGGCAGGAAAGAGGGG-3' (SEQ ID No. 29);
*mlnfaRl* forward, 5'-CACGGTCGCTGTAGAAGTAAAG-3' (SEQ ID No. 30); *mlnfaRl* reverse, 5'-TCTCCTCCTCTTCGTTGGAATA-3' (SEQ ID No. 31);
*mInfgR1* forward, 5'- CTTGAACCCTGTCGTATGCTGG -3' (SEQ ID No. 32); *mInfgR1* reverse, 5'-TTGGTGCAGGAATCAGTCCAGG -3' (SEQ ID No. 33);
*mCxCl10* forward, 5'-GAATCCGGAATCTAAGACCATCAA-3' (SEQ ID No. 34); *mCxCl10* reverse, 5'-GTGCGTGGCTTCACTCCAGT-3' (SEQ ID No. 35);
*mPD-L1* forward, 5'-TGCGGACTACAAGCGAATCACG-3' (SEQ ID No. 36); *mPD-L1* reverse, 5'-CTCAGCTTCTGGATAACCCTCG-3' (SEQ ID No. 37);
*mStat1* forward, 5'-GCCTCTCATTGTCACCGAAGAAC-3' (SEQ ID No. 38); *mStat1* reverse, 5'-TGGCTGACGTTGGAGATCACCA -3' (SEQ ID No. 39);
*mStat2* forward, 5'-GAACCAACTCTCCATTGCCTGG-3' (SEQ ID No. 40); *mStat2* reverse, 5'-CGTAAGAGGAGAACTGCCAGCT-3' (SEQ ID No. 41);
*mIgs-15* forward, 5'-ACCCTTTCCAGTCTGGGTCT-3' (SEQ ID No. 42); *mIgs-15* reverse, 5'-TCGCTGCAGTTCTGTACCAC-3' (SEQ ID No. 43);
*mMHC-I* forward, 5'- GCTGGTGAAGCAGAGAGACTCAG-3' (SEQ ID No. 44); *MHC-I* reverse, 5'- GGTGACTTTATCTTCAGGTCTGCT-3' (SEQ ID No. 45);
*mB-actin* forward, 5'-GTGACGTTGACATCCGTAAAG-3' (SEQ ID No. 46); *mB-actin* reverse, 5'-GCCGGACTCATCGTACTCC-3' (SEQ ID No. 47);
*hCited2* forward, 5'-TGCCGCCCAATGTCATAGACAC-3' (SEQ ID No. 48); *hCited2* reverse, 5'-CAGCTCCTTGATGCGGTCCAAA-3' (SEQ ID No. 49);
*hB-actin* forward, 5'-CAAGGCCAACCGCGAGAAGATGAC-3' (SEQ ID No. 50); *hB-actin* reverse, 5'-GCCAGAGGCGTACAGGGATAGCACA-3' (SEQ ID No. 51).

### Example 1: Loss of CITED2 compromises survival of Lgr5⁺ intestinal stem cells

First the inventors examined the distribution pattern of CITED2 expression in intestinal crypts to determine whether CITED2 would potentially impact intestinal epithelial cell sternness and tissue homeostasis. Immunoblot analysis of fractionated crypts and villi indicated that CITED2 expression was restricted to the crypt compartment (Figure 1A). While immunohistochemical staining confirmed prominent nuclear CITED2 expression at the crypt base in both Lgr5+ and Paneth cells gradually decreasing towards the transit-amplifying compartment (Figure 1B). Restricted CITED2 expression to the stem cell compartment was also recapitulated in small intestinal organoid cultures derived from Lgr5-GFP-DTR mice (19, 20).

Next, the CITED2 function was explored by generating mice with an intestinal epithelial cell (IEC) specific deletion of *Cited2* by crossing floxed *Cited2 (Cited2*^{*F*/}*^{F})* mice (21) with tamoxifen-inducible *villin-*Cre^{ERT2} mice (22). 4 days after tamoxifen administration IEC-restricted *Cited2* loss led to an altered intestinal architecture characterized by the appearance of abundant apoptotic bodies in the crypt and crypt elongation (Figures 1C and 1D). Cleaved caspase 3 immunostaining confirmed an increased number of apoptotic cells mainly in the ISC niche (Figures 1E and 1F), which was accompanied by reduced OLFM4 expression (Figure 1G) thus indicating loss of crypt bottom stem cells following acute *Cited2* deletion.

In situ hybridization confirmed loss of *Lgr5* mRNA (Figure 1H) and immunofluorescent staining of GFP revealed a marked reduction of Lgr5+ cells in *Lgr5-GFP-DTR;Cited2^{ΔIEC}* compound mutants (Figure 1I). Moreover, tracing of pre-existing Lgr5+ ISCs and their progeny in *Lgr5-EGFP-Cre^{ERT2};Rosa26-tdTomato;Cited2^{ΔIEC}* compound mice showed a lack of tdTomato⁺ cells throughout the crypt-villus axis when compared to control sections.

However, when the inventors examined *Cited2^{ΔIEC}* mice 10 days after tamoxifen administration no histological differences were observed any longer and all crypts showed regular *Olfm4* expression as well as homogenous CITED2 staining demonstrating that CITED2-deficient crypts had been displaced and repopulated by non-recombined CITED2-proficient stem cells. Similar to intestinal crypts in vivo, CITED2 was also critical for *ex vivo* organoid growth. 4-hydroxytamoxifen induced *Cited2* deletion in established organoid cultures led to impaired branching and decreased organoid survival (Figures 1J and 1K) preventing passaging of *Cited2-*deficient cells. Quantitative PCR at day 3 confirmed that *Cited2* loss was associated with reduced *Lgr5* gene expression.

Moreover, there was strong depletion of Lgr5⁺ ISCs, assessed by reduced GFP staining, in intestinal organoids derived from *Lgr5-EGFP* expressing *Cited2*^{*Δ*IEC} mice after tamoxifen administration. *Cited2* depletion was associated with induction of cleaved caspase-3 and p21 (Figure 1L). In agreement with a Tp53-dependent upregulation of these, Cited2 deletion triggered Tp53Ser15 phosphorylation (Figure 1L) suggesting induction of DNA damage (23, 24, 25). *Cited2*-deletion led to a pronounced γH2AX upregulation in organoids, which could also be detected in crypt epithelial cells from *Cited2*^{*Δ*IEC} mice although *Cited2* was only partially deleted (Figures 1M and 1N). Collectively, these data demonstrated that CITED2 plays an essential role for Lgr5⁺ intestinal stem cell survival by preventing DNA damage.

### Example 2: CITED2 marks mitotic machinery and prevents mitotic defects in intestinal epithelia

For the investigation how *Cited2* suppresses genomic damage, the inventors performed RNA-sequence analysis of whole mucosa small intestinal samples 18 hours post tamoxifen administration. This represents a time point when *Cited2* is fully deleted but caspase 3⁺ crypt epithelial cells have not formed yet in *Cited2*^{*Δ*IEC} mice. Transcriptomic analysis revealed distinct gene-clusters in *Cited2*-deficient mucosa that were associated with ribosomal RNA processing and particularly cell cycle progression linked to separation of sister chromatid segregation, M (mitosis) phase, mitotic prometaphase, cell cycle check points and G2/M transition (Figures 2A and 2B). Most of the differentially regulated genes within the M cluster were mainly associated with centrosome biogenesis (*Cep56, Cep57, Cep70, Cep135, Cep290*), microtubule spindle organization (*Nedd1, Kif23, Kif20a, Kif2a, Kif18a, Tubb2a, Tubb4a*)*,* cohesion (*smc2, smc4*), centromere (*Cenpa, Cenph, Cenpk, Cenpn*), and kinetochores (*Bub1, Mad2l1*) suggesting that CITED2 regulates establishment of early mitosis (Figure 2B).

In line with these transcriptomic changes, the inventors detected frequent appearance of chromatin/anaphase bridges, chromosome misalignments and cells with phenotypic characteristics of mitotic catastrophe such as hyper-condensed chromatin aggregates in crypts from *Cited2*^{*Δ*IEC} but not control mice (Figures 2C and 2D) indicating that CITED2 is required for the maintenance of mitotic fidelity in the intestinal stem cell compartment.

Next, the inventors silenced CITED2 in immortalized mouse intestinal epithelial cells (Mode-K cells) to functionally examine how CITED2 promotes proper mitosis. Like intestinal crypt cells *in vivo,* doxycyline-induced knockdown of CITED2 led to upregulation of γH2AX and cleaved caspase 3, which was associated with a reduced colony formation. Cell cycle analysis on asynchronous CITED2-silenced cells demonstrated a substantial increase in the G2/M population with a concomitant reduction of cells in G1 phase (Figure 2E).

A block in mitosis was verified by enhanced phospho-histone H3 and Cyclin B 1 staining. In stark contrast, the majority of CITED2-knockdown mitotic cells were found to be poorly labelled for phospho-MPM2, which specifically recognizes phosphorylated proteins that are associated with structural components of the mitotic apparatus (26). Importantly, the inventors noticed that subcellular localization of CITED2 itself changed during cell cycle. During interphase CITED2 was diffusely distributed in the nucleus and largely present in the nucleoplasm but excluded from the nucleolus where nucleoin (NCL) was localized (Figure 2F). However, upon entry into mitosis CITED2 showed distinct accumulation at microtubule organizing centers (MTOCs) and subsequently at the intercellular bridge between the two daughter cells prior to cytokinesis indicating a dynamic localization pattern during mitosis (Figure 2F).

The localization pattern of CITED2 indicated an association with spindle microtubules which suggested an active involvement of CITED2 in early mitosis independently of its function as a transcriptional coactivator (Figure 2G). Knockdown of CITED2 in Mode-K cells culminated in a prometaphase arrest, associated with severe chromosome congression defects (Figures 2H and 2I). BUB 1 kinetochore staining verified the lack of bi-oriented chromosome line-up at the equatorial plane and showed dispersed chromosome positions around the metaphase plate in *Cited2*-deficient cells reminiscent of prometaphase cell phase (Figure 2J).

In line with this, Cited2-deficient mitotic cells revealed various mitotic defects including lagging chromosomes, chromosome bridges, and multipolar mitosis (Figures 2K and 2L). Moreover, multinucleated cells showing polyploid chromosomes indicated cytokinesis failure and induction of mitotic catastrophe in CITED2 knock-down cells. Collectively, these findings provide evidence that CITED2 is an essential regulatory element of intestinal epithelial cell mitosis by controlling both transcription of genes driving cell cycle progression as well as by organizing and establishing MTOC formation.

### Example 3: CITED2 promotes centrosome maturation and bipolar spindle formation

In eukaryotic cells, centrosomes are the key MTOCs, which are crucial for bipolar spindle assembly and faithful chromosome segregation in cell division (27). The recruitment of CITED2 to MTOCs and mitotic spindles raised the possibility that CITED2 might be involved in centrosome dynamics. While during interphase there was no evidence for an interaction of the integral centrosome component Pericentrin and CITED2, these two clearly colocalized at the onset of mitosis (Figure 3A). Accordingly, loss of *Cited2* led to centrosome separation defects (Figures 3A and 3B).

In addition to unseparated centrosomes, an increased centrosome number could be detected in CITED2-deficient Mode-K cells confirming its essential role for centrosome dynamics (Figure 3C). Centrosomes not only nucleate but also organize microtubules of the mitotic spindle. Unseparated centrosomes in conjunction with chromosome misalignment and unbalanced segregation in CITED2 shRNA-expressing cells point out an aberrant mitotic spindle assembly. In fact, CITED2 knock-down cells displayed mainly disorganized monopolar mitotic spindles, which were accumulated at the center of the mitotic cells in comparison to normal bipolar mitotic spindles found in control cells (Figure 3D).

To determine whether the short/monopolar spindles observed after CITED2 knockdown resulted from a failure in centrosome separation or compromised microtubule nucleation potential, a microtubule regrowth assay was performed. Following cold mediated microtubule depolymerization, both control and CITED2 depleted cells displayed robust microtubule nucleation indicating that cells with reduced CITED2 expression cannot establish bipolar spindle due to centrosome pole movement defects.

Bipolar nucleation of mitotic spindles at centrosomes requires a crucial step known as centrosome maturation that includes the centrosome growth and accumulation of pericentriolar material (PCM) such as Cep192, γTuRC, and mitotic kinases including Polo-like kinase 1 (PLK1) and Aurora A (28), the latter being essential for kinesin-5 (EG5)-mediated centrosome separation, bipolar spindle formation, and equal centrosome/centriole segregation into daughter cells (29).

Considering the decreased MPM2 phosphorylation, the inventors examined the activation of PLK1 and aurora kinases. Immunofluorescence analysis indicated a diminished phosphorylation of both PLK1 and pan-Aurora in mitotic cells when CITED2 expression is silenced (Figure 3E). Additionally, the immunoblot analysis confirmed that CITED2 knockdown cells could not sustain Aurora phosphorylation, which acts upstream of PLK1 in the PCM assembly pathway, when cells were arrested at the G1/S transition and then released to allow synchronous progression through S, G2, and M (Figure 3F). In line with these findings, EG5 could be detected at the spindle poles during metaphase and anaphase in CITED2-proficient cells, whereas CITED2 knockdown cells displayed diminished and disorganized EG5 localization only at the center of mitotic cells (Figure 3G). Together these data indicate that CITED2 loss impairs activation of PLK1 and aurora kinases ultimately causing defects in centrosome maturation and bipolar spindle formation which leads to an arrest in prometaphase.

### Example 4: Interaction of CITED2 and Cdk11b^{p58} is essential for mitosis

Prompted by the unexpected localization of CITED2 at centrosomes, the inventors reasoned that CITED2 may impact centrosome maturation independently of its well-known function as a transcriptional co-activator. Therefore, the inventors performed mass spectrometry analysis to identify putative CITED2 binding proteins known to be essential for mitotic fidelity. For this purpose, the inventors applied SILAC labeling of Mode-K cells and performed proteomic analysis on either G1/S- (interphase) or G2/M- (mitosis) phase arrested samples to identify the respective CITED2-interactome (Figure 4A). Immunoblot analysis of phospho-histone H3 was used to verify entry of cells into mitosis after double thymidine-mediated cell cycle block at G1/S phase. The inventors were able to validate with their experimental setup previously described CITED2 interactors from HEK293T cells such as tubulin alpha/beta, nucleolin, heat shock protein 90, pyruvate kinase, splicing factor 3b, dynein, and interleukin enhancer binding factor 3 (30).

Importantly, the inventors discovered several candidates specifically binding to CITED2 during G2/M phase. The inventors focused on cyclin-dependent kinase 1 1b (Cdk11b) as it is required for centrosome maturation and bipolar spindle formation (31). *Cdk11b* encodes a 110-kDa protein (Cdk11b^{p110}) throughout the cell cycle and a 58-kDa protein (Cdk11b^{p58}) that is specifically translated from an internal ribosome entry site sequence during G2/M (32, 33). The inventors introduced into CITED2 proficient and deficient Mode-K cells the Cdk11b^{p58} isoform in a doxycycline-dependent manner to validate their interactome results and examine involvement of Cdk11b in CITED2 knockdown-driven mitosis related phenotypes. Notably, ectopic expression of Cdk11b^{p58} did not influence the cell cycle distribution in CITED2 proficient Mode-K cells. Confocal microscopy indicated colocalization of CITED2 and Cdk11b during mitosis and immunoblot analysis confirmed binding of CITED2 to Cdk11b^{p58} in Cdk11b^{p58}-overexpressing Mode-K cells (Figures 4B and 4C).

Microscopic analysis showed that introduction of Cdk11b^{p58} expression in *Cited2*-deficient Mode-K cells prevented the increased number of mitotic round cells and excessive phospho-histone H3 accumulation in nocodazole arrested *Cited2*-deficient cells. Importantly, the inventors found that forced Cdk11b^{p58} expression in asynchronous CITED2 knockdown cells restored bipolar spindle formation and corrected unaligned chromosome alignment defects, which resulted in rescue of early prometaphase arrest (Figure 4D). Consistently, the inventors found proper centrosome separation in Cdk11b^{p58}-overexpressing CITED2 RNAi mitotic cells suggesting normal centrosome maturation (Figures 4E and 4F). Immunofluorescence staining for multiple markers that regulates centrosome movement and maturation showed that ectopic Cdk11b^{p58} expression normalized expression of pMPM2, Aurora/PLK1 phosphorylation and their common downstream target EG5 in CITED2-silenced cells demonstrating presence of functional centrosomes and normal mitotic progression (Figures 4G and 4H).

Notably, there were no changes in localization of Aurora-A, Aurora-B and PLK proteins in CITED2 knockdown mitotic cells suggesting that activity of these proteins rather than their localization is modulated via Cdk11b^{p58}. Finally, the inventors found that normalization of aberrant mitosis in CITED2 knockdown cells via forced expression of Cdk11b^{p58} resulted in significantly reduced DDR and apoptosis confirming that the blockade of mitotic progression is the underlying reason for genomic damage and mitotic catastrophe-associated cell death (Figure 4H). Altogether these data indicate that CITED2-driven Cdk11b^{p58} expression mediates Aurora-PLK1-EG5 key signaling cascade in the regulation of early mitosis to control centrosome maturation and bipolar spindle assembly, which finally contribute to chromosome stability and faithful cell division.

### Example 5: Downregulation of CITED2 causes tumor regression by blocking cell cycle progression and stimulating anti-tumor immunity

Considering the strong impact on mitosis, the inventors reasoned that CITED2 represents a promising target for cancer therapy. The inventors silenced CITED2 shortly in human SW48 CRC cells with a gapmer antisense oligonucleotide. Like in Mode-K cells, acute loss of CITED2 expression in human SW48 cells severely compromised their cell survival, which was associated with defective bipolar mitotic spindle formation and subsequent chromosome alignment (Figures 5A and 5B).

Similarly, long-term knockdown of CITED2 in SW48 cells demonstrated elevated caspase-3 and p21 expression followed by diminished clonogenic growth. To assess the impact of CITED2 loss *in vivo,* the inventors challenged *Rosa26-Cre^{ERT2};Cited2*^{*F*/*F*} and *Rosa26-Cre^{ERT2}* control mice with the carcinogen azoxymethane (AOM) followed by three cycles of 2% dextran sulfate sodium (DSS) to induce orthotopic colonic tumours that were characterized by marked CITED2 upregulation. The inventors induced CITED2 deletion in established tumours by administering tamoxifen interrupted by one week. Following the second cycle of tamoxifen the inventors detected only tumor remnants in *Rosa26-Cre^{ERT2};Cited2*^{Δ/Δ} mice whereas the inventors macroscopically did not observe any overt impact of tamoxifen on wild type tumors (Figure 5C). Further histological analysis confirmed a significant reduction in tumor number and size after CITED2 ablation (Figures 5D-F).

Immunohistochemical analysis revealed marked reduction in cell proliferation and increased apoptosis in residual CITED2-deleted tumors. Interestingly, the inventors found a profound accumulation of CD3⁺ T cells in CITED2-deleted tumors, which was accompanied by a marked PD-L1 expression in CITED2-deficient tumor epithelia. Moreover, RNA analysis revealed elevated gene expression of *Ifng, Irf7, InfaR1, Isg15, Cxcl10, Mx2,* apart from *Pdl1* in both *Cited2^{ΔIEC}* organoids and CITED2-deficient Mode-K cells indicating an activation of interferon signaling. Replication stress and chromosome mis-segregation can activate cGAS/STING signaling thereby triggering type I interferon production (34). To examine whether the observed interferon response in CITED2-deficient Mode-K cells was linked to cGAS/STING activation, the inventors treated CITED2^{wt} and CITED2^{KD} Mode-K cells with specific STING inhibitors C176 and C178 and found indeed a blunted induction of interferon target genes in CITED2^{KD} cells upon STING blockade. These data suggested that CITED2-deficient tumors induce an anti-tumoral immune response possibly rendering them amenable to immune checkpoint blockade.

Therefore, the inventors engineered murine APT (*APC*^{*Δ*/}*^{Δ}, TP53*^{*Δ*/*Δ*} and *TGFER2*^{*Δ*/*Δ*}) tumor organoids with doxycycline-inducible control shRNA or shRNAs targeting CITED2 and injected them subcutaneously into C57BL/6J mice. Once tumors reached visible size, the iventors initiated doxycycline treatment and observed a significant suppression of APT^{Cited2KD} tumors (Figure 5G). While a-PD-1 treatment led to tumor stasis in APT^{wt} tumors, essentially no Ki-67⁺ tumor cells could be detected in APT^{Cited2KD} tumors anymore (Figure 5I). Moreover, a-PD-1 treated APT^{Cited2KD} tumors demonstrated a massive infiltration of CD8⁺ T cells (Figure 5I) indicating a stronger anti-tumor immunity.

To further prove that CITED2-deficient tumor cell clearance was in part dependent on immune cells, the inventors injected APT cells s.c. into immunodeficient NSG mice. While loss of CITED2 still had a pronounced effect on blocking tumor growth and tumor cell proliferation, the number of EpCAM⁺/CITED2^{KD} tumor epithelia was significant lower in immune competent mice confirming the contribution of adaptive immune cells in control of CITED2^{KD} tumors (Figure 5M). Collectively, these data suggested that loss of CITED2 leads to a direct cell autonomous as well as an immune mediated tumor suppression.

### References

1. Barker, N., et al. (2007). Identification of stem cells in small intestine and colon by marker gene Lgr5. Nature 449, 1003-1007. 10.1038/nature06196.
2. Potten, C.S., and Loeffler, M. (1990). Stem cells: attributes, cycles, spirals, pitfalls and uncertainties. Lessons for and from the crypt. Development 110, 1001-1020.
3. Schepers, A.G., Vries, R., van den Born, M., van de Wetering, M., and Clevers, H. (2011). Lgr5 intestinal stem cells have high telomerase activity and randomly segregate their chromosomes. EMBO J 30, 1104-1109. 10.1038/emboj.2011.26.
4. Tao, S., Tang, D., Morita, Y., Sperka, T., Omrani, O., Lechel, A., Sakk, V., Kraus, J., Kestler, H.A., Kuhl, M., and Rudolph, K.L. (2015). Wnt activity and basal niche position sensitize intestinal stem and progenitor cells to DNA damage. EMBO J 34, 624-640. 10.15252/embj.201490700.
5. Beumer, J., and Clevers, H. (2024). Hallmarks of sternness in mammalian tissues. Cell Stem Cell 31, 7-24. 10.1016/j.stem.2023.12.006.
6. Bhattacharya, S., Michels, C.L., Leung, M.K., Arany, Z.P., Kung, A.L., and Livingston, D.M. (1999). Functional role of p35srj, a novel p300/CBP binding protein, during transactivation by HIF-1. Genes Dev 13, 64-75.
7. Preis, J.I., Wise, N., Solloway, M.J., Harvey, R.P., Sparrow, D.B., and Dunwoodie, S.L. (2006). Generation of conditional Cited2 null alleles. Genesis 44, 579-583. 10.1002/dvg.20251.
8. Chou, Y.T., and Yang, Y.C. (2006). Post-transcriptional control of Cited2 by transforming growth factor beta. Regulation via Smads and Cited2 coding region. J Biol Chem 281, 18451-18462. 10.1074/jbc.M601720200.
9. Kranc, K.R., Bamforth, S.D., Braganca, J., Norbury, C., van Lohuizen, M., and Bhattacharya, S. (2003). Transcriptional coactivator Cited2 induces Bmi1 and Mel18 and controls fibroblast proliferation via Ink4a/ARF. Mol Cell Biol 23, 7658-7666.
10. Pritsker, M., Ford, N.R., Jenq, H.T., and Lemischka, I.R. (2006). Genomewide gain-of-function genetic screen identifies functionally active genes in mouse embryonic stem cells. Proc Natl Acad Sci U S A 103, 6946-6951. 10.1073/pnas.0509861103.
11. Kranc, K.R., Oliveira, D.V., Armesilla-Diaz, A., Pacheco-Leyva, I., Catarina Matias, A., Luisa Escapa, A., Subramani, C., Wheadon, H., Trindade, M., Nichols, J., et al. (2015). Acute loss of Cited2 impairs Nanog expression and decreases self-renewal of mouse embryonic stem cells. Stem Cells 33, 699-712. 10.1002/stem.1889.
12. Li, Q., Ramirez-Bergeron, D.L., Dunwoodie, S.L., and Yang, Y.C. (2012). Cited2 gene controls pluripotency and cardiomyocyte differentiation of murine embryonic stem cells through Oct4 gene. J Biol Chem 287, 29088-29100. 10.1074/jbc.M112.378034.
13. Bamforth, S.D., Braganca, J., Eloranta, J.J., Murdoch, J.N., Marques, F.I., Kranc, K.R., Farza, H., Henderson, D.J., Hurst, H.C., and Bhattacharya, S. (2001). Cardiac malformations, adrenal agenesis, neural crest defects and exencephaly in mice lacking Cited2, a new Tfap2 coactivator. Nat Genet 29, 469-474. 10.1038/ng768.
14. Weninger, W.J., Lopes Floro, K., Bennett, M.B., Withington, S.L., Preis, J.I., Barbera, J.P., Mohun, T.J., and Dunwoodie, S.L. (2005). Cited2 is required both for heart morphogenesis and establishment of the left-right axis in mouse development. Development 132, 1337-1348. 10. 1242/dev.01696.
15. Kranc, K.R., Schepers, H., Rodrigues, N.P., Bamforth, S., Villadsen, E., Ferry, H., Bouriez-Jones, T., Sigvardsson, M., Bhattacharya, S., Jacobsen, S.E., and Enver, T. (2009). Cited2 is an essential regulator of adult hematopoietic stem cells. Cell Stem Cell 5, 659-665. 10.1016/j.stem.2009.11.001.
16. Lawson, H., van de Lagemaat, L.N., Barile, M., Tavosanis, A., Durko, J., Villacreces, A., Bellani, A., Mapperley, C., Georges, E., Martins-Costa, C., et al. (2021). CITED2 coordinates key hematopoietic regulatory pathways to maintain the HSC pool in both steady-state hematopoiesis and transplantation. Stem Cell Reports 16, 2784-2797. 10.1016/j.stemcr.2021.10.001. 30
17. Zhou, Z., Akinbiyi, T., Xu, L., Ramcharan, M., Leong, D.J., Ros, S.J., Colvin, A.C., Schaffler, M.B., Majeska, R.J., Flatow, E.L., and Sun, H.B. (2010). Tendon-derived stem/progenitor cell aging: defective self-renewal and altered fate. Aging Cell 9, 911-915. 10.1111/j. 1474-9726.2010.00598.x.
18. Keyes, B.E., Segal, J.P., Heller, E., Lien, W.H., Chang, C.Y., Guo, X., Oristian, D.S., Zheng, D., and Fuchs, E. (2013). Nfatc1 orchestrates aging in hair follicle stem cells. Proc Natl Acad Sci U S A 110, E4950-4959. 10.1073/pnas.1320301110.
19. Tian, H., Biehs, B., Warming, S., Leong, K.G., Rangell, L., Klein, O.D., and de Sauvage, F.J. (2011). A reserve stem cell population in small intestine renders Lgr5-positive cells dispensable. Nature 478, 255-259. 10.1038/nature10408.
20. de Sousa e Melo, F., Kurtova, A.V., Harness, J.M., Kljavin, N., Hoeck, J.D., Hung, J., Anderson, J.E., Storm, E.E., Modrusan, Z., Koeppen, H., et al. (2017). A distinct role for Lgr5(+) stem cells in primary and metastatic colon cancer. Nature 543, 676-680. 10.1038/nature21713.
21. MacDonald, S.T., Bamforth, S.D., Chen, C.M., Farthing, C.R., Franklyn, A., Broadbent, C., Schneider, J.E., Saga, Y., Lewandoski, M., and Bhattacharya, S. (2008). Epiblastic Cited2 deficiency results in cardiac phenotypic heterogeneity and provides a mechanism for haploinsufficiency. Cardiovasc Res 79, 448-457. 10.1093/cvr/cvn101.
22. el Marjou, F., Janssen, K.P., Chang, B.H., Li, M., Hindie, V., Chan, L., Louvard, D., Chambon, P., Metzger, D., and Robine, S. (2004). Tissue-specific and inducible Cre-mediated recombination in the gut epithelium. Genesis 39, 186-193. 10.1002/gene.20042.
23. Espinosa, J.M. (2008). Mechanisms of regulatory diversity within the p53 transcriptional network. Oncogene 27, 4013-4023. 10.1038/onc.2008.37. 24. Kruse, J.P., and Gu, W. (2009). Modes of p53 regulation. Cell 137, 609-622. 10.1016/j.cell.2009.04.050.
25. Siliciano, J.D., Canman, C.E., Taya, Y., Sakaguchi, K., Appella, E., and Kastan, M.B. (1997). DNA damage induces phosphorylation of the amino terminus of p53. Genes Dev 11, 3471-3481.
26. Ding, M., Feng, Y., and Vandre, D.D. (1997). Partial characterization of the MPM-2 phosphoepitope. Exp Cell Res 231, 3-13. 10.1006/excr.1996.3439.
27. Bornens, M. (2021). Centrosome organization and functions. Curr Opin Struct Biol 66, 199-206. 10.1016j.sbi.2020.11.002.
28. Cabral, G., Laos, T., Dumont, J., and Dammermann, A. (2019). Differential Requirements for Centrioles in Mitotic Centrosome Growth and Maintenance. Dev Cell 50, 355-366 e356. 10.1016/j.devcel.2019.06.004.
29. Joukov, V., Walter, J.C., and De Nicolo, A. (2014). The Cep192-organized aurora A-Plk1 cascade is essential for centrosome cycle and bipolar spindle assembly. Mol Cell 55, 578-591. 10.1016/j.molcel.2014.06.016.
30. Shin, S.H., Lee, G.Y., Lee, M., Kang, J., Shin, H.W., Chun, Y.S., and Park, J.W. (2018). Aberrant expression of CITED2 promotes prostate cancer metastasis by activating the nucleolin-AKT pathway. Nat Commun 9, 4113. 10.1038/s41467-018-06606-2.
31. Petretti, C., Savoian, M., Montembault, E., Glover, D.M., Prigent, C., and Giet, R. (2006). The PITSLRE/CDK11p58 protein kinase promotes centrosome maturation and bipolar spindle formation. EMBO Rep 7, 418-424. 10.1038/sj.embor.7400639.
32. Cornelis, S., Bruynooghe, Y., Denecker, G., Van Huffel, S., Tinton, S., and Beyaert, R. (2000). Identification and characterization of a novel cell cycle-regulated internal ribosome entry site. Mol Cell 5, 597-605. 10.1016/s1097-2765(00)80239-7.
33. Hu, D., Valentine, M., Kidd, V.J., and Lahti, J.M. (2007). CDK11(p58) is required for the maintenance of sister chromatid cohesion. J Cell Sci 120, 2424-2434. 10.1242/jcs.007963.
34. Long, Z.J., Wang, J.D., Xu, J.Q., Lei, X.X., and Liu, Q. (2022). cGAS/STING cross-talks with cell cycle and potentiates cancer immunotherapy. Mol Ther 30, 1006-1017.
10.1016/j.ymthe.2022.01.044. 35. Barr, A.R., and Gergely, F. (2007). Aurora-A: the maker and breaker of spindle poles. J Cell Sci 120, 2987-2996. 10.1242/jcs.013136.
36. Loyer, P., and Trembley, J.H. (2020). Roles of CDK/Cyclin complexes in transcription and pre-mRNA splicing: Cyclins L and CDK11 at the cross-roads of cell cycle and regulation of gene expression. Semin Cell Dev Biol 107, 36-45. 10.1016/j.semcdb.2020.04.016.
37. Hluchy, M., Gajduskova, P., Ruiz de Los Mozos, I., Rajecky, M., Kluge, M., Berger, B.T., Slaba, Z., Potesil, D., Weiss, E., Ule, J., et al. (2022). CDK11 regulates pre-mRNA splicing by phosphorylation of SF3B1. Nature 609, 829-834. 10.1038/s41586-022-05204-z.
38. Feng, Y., Qi, W., Martinez, J., and Nelson, M.A. (2005). The cyclin-dependent kinase 11 interacts with 14-3-3 proteins. Biochem Biophys Res Commun 331, 1503-1509. 10.1016/j.bbrc.2005.04.078.
39. Aitken, A. (2006). 14-3-3 proteins: a historic overview. Semin Cancer Biol 16, 162-172. 10.1016/j.semcancer.2006.03.005.
40. Bose, A., and Dalal, S.N. (2019). 14-3-3 proteins mediate the localization of Centrin2 to centrosome. J Biosci 44.
41. Mukhopadhyay, A., Sehgal, L., Bose, A., Gulvady, A., Senapati, P., Thorat, R., Basu, S., Bhatt, K., Hosing, A.S., Balyan, R., et al. (2016). 14-3-3gamma Prevents Centrosome Amplification and Neoplastic Progression. Sci Rep 6, 26580. 10.1038/srep26580.
42. Kasahara, K., Goto, H., Izawa, I., Kiyono, T., Watanabe, N., Elowe, S., Nigg, E.A., and Inagaki, M. (2013). PI 3-kinase-dependent phosphorylation of Plk1-Ser99 promotes association with 14-3-3gamma and is required for metaphase-anaphase transition. Nat Commun 4, 1882. 10.1038/ncomms2879.
43. Hu, C., Zhang, Y., Tang, K., Luo, Y., Liu, Y., and Chen, W. (2017). Downregulation of CITED2 contributes to TGFbeta-mediated senescence of tendon-derived stem cells. Cell Tissue Res 368, 93-104. 10.1007/s00441-016-2552-1.
44. Korthuis, P.M., Berger, G., Bakker, B., Rozenveld-Geugien, M., Jaques, J., de Haan, G., Schuringa, J.J., Vellenga, E., and Schepers, H. (2015). CITED2-mediated human hematopoietic stem cell maintenance is critical for acute myeloid leukemia. Leukemia 29, 625-635. 10.103 8/leu.2014.259.
45. Vooijs, M., Jonkers, J., and Berns, A. (2001). A highly efficient ligand-regulated Cre recombinase mouse line shows that LoxP recombination is position dependent. EMBO Rep 2, 292-297. 10.1093/embo-reports/kve064.
46. Diamanti, M.A., Gupta, J., Bennecke, M., De Oliveira, T., Ramakrishnan, M., Braczynski, A.K., Richter, B., Beli, P., Hu, Y., Saleh, M., et al. (2017). IKKalpha controls ATG16L1 degradation to prevent ER stress during inflammation. J Exp Med 214, 423-437. 10.1084/jem.20161867.
47. Greten, F.R., Eckmann, L., Greten, T.F., Park, J.M., Li, Z.W., Egan, L.J., Kagnoff, M.F., and Karin, M. (2004). IKKbeta links inflammation and tumorigenesis in a mouse model of colitis-associated cancer. Cell 118, 285-296. 10.1016/j.cell.2004.07.013.
48. De Oliveira, T., Ramakrishnan, M., Diamanti, M.A., Ziegler, P.K., Brombacher, F., and Greten, F.R. (2019). Loss of Stat6 affects chromatin condensation in intestinal epithelial cells causing diverse outcome in murine models of inflammation-associated and sporadic colon carcinogenesis. Oncogene 38, 1787-1801. 10.1038/s41388-018-0551-2.
49. Sato, T., Vries, R.G., Snippert, H.J., van de Wetering, M., Barker, N., Stange, D.E., van Es, J.H., Abo, A., Kujala, P., Peters, P.J., and Clevers, H. (2009). Single Lgr5 stem cells build crypt-villus structures in vitro without a mesenchymal niche. Nature 459, 262-265. 10.1038/nature07935.
50. Fellmann, C., Hoffmann, T., Sridhar, V., Hopfgartner, B., Muhar, M., Roth, M., Lai, D.Y., Barbosa, I.A., Kwon, J.S., Guan, Y., et al. (2013). An optimized microRNA backbone for effective single-copy RNAi. Cell Rep 5, 1704-1713. 10.1016/j.celrep.2013.11.020.
51. Fellmann, C., Zuber, J., McJunkin, K., Chang, K., Malone, C.D., Dickins, R.A., Xu, Q., Hengartner, M.O., Elledge, S.J., Hannon, G.J., and Lowe, S.W. (2011). Functional identification of optimized RNAi triggers using a massively parallel sensor assay. Mol Cell 41, 733-746. 10.1016/j.molcel.2011.02.008.
52. Chen, E.Y., Tan, C.M., Kou, Y., Duan, Q., Wang, Z., Meirelles, G.V., Clark, N.R., and Ma'ayan, A. (2013). Enrichr: interactive and collaborative HTML5 gene list enrichment analysis tool. BMC Bioinformatics 14, 128. 10.1186/1471-2105-14-128.
53. Kuleshov, M.V., Jones, M.R., Rouillard, A.D., Fernandez, N.F., Duan, Q., Wang, Z., Koplev, S., Jenkins, S.L., Jagodnik, K.M., Lachmann, A., et al. (2016). Enrichr: a comprehensive gene set enrichment analysis web server 2016 update. Nucleic Acids Res 44, W90-97. 10.1093/nar/gkw377.
54. Yu, G., and He, Q.Y. (2016). ReactomePA: an R/Bioconductor package for reactome pathway analysis and visualization. Mol Biosyst 12, 477-479. 10.1039/c5mb00663e.
55. Wilke, A.C., Doebele, C., Zindel, A., Lee, K.S., Rieke, S.A., Ceribelli, M., Comoglio, F., Phelan, J.D., Wang, J.Q., Pikman, Y., et al. (2022). SHMT2 inhibition disrupts the TCF3 transcriptional survival program in Burkitt lymphoma. Blood 139, 538-553. 10.1182/blood.2021012081.
56. Tyanova, S., Temu, T., and Cox, J. (2016). The MaxQuant computational platform for mass spectrometry-based shotgun proteomics. Nat Protoc 11, 2301-2319. 10.1038/nprot.2016.136.

## Claims

1. A method for identifying a compound that modulates the mitotic cell cycle in cancer cells by modulating at least one of the expression, the amount, the stability and/or the biological activity of the protein CITED2 and/or CITED2 containing protein complexes in a mammalian cell, comprising the steps of:
a) contacting at least one of CITED2 or a functional fragment thereof and/or a cell expressing CITED2 or the functional fragment thereof with at least one compound that potentially modulates at least one of the expression, the amount, the stability and/or the biological activity of the protein CITED2 in the mammalian cell,
b) identifying a modulation of at least one of the expression, the stability and/or the biological activity of the protein CITED2 in the presence of the at least one compound, when compared to the absence of said at least one compound, and
c) identifying a compound of step b) as modulating, in particular as specifically modulating, the mitotic cell cycle in the cancer cells based on said modulation as identified in step b).

2. The method according to claim 1, wherein said modulation is selected from a decrease or an increase of the expression, the amount, the stability and/or the biological activity of the protein CITED2 in a mammalian cell, preferably an inhibition of the expression, the amount, the stability and/or the biological activity of the protein CITED2, wherein the biological activity is preferably selected from the interaction of CITED2 with at least one molecule selected from the group consisting of CDK11b^{p58}, splicing factor 3B subunit 1 (SF3B1), and 14-3-3γ.

3. A method for identifying a compound that modulates the mitotic cell cycle in cancer cells, by modulating at least one of the interaction of CITED2 with at least one molecule selected from the group consisting of CDK11b^{p58}, splicing factor 3B subunit 1 (SF3B1), and 14-3-3γ in a mammalian cell, comprising the steps of:
a) contacting at least one of CITED2, CDK11b^{p58}, splicing factor 3B subunit 1 (SF3B1) and/or 14-3-3γ binding fragment of CITED2, a cell expressing CITED2 and/or a cell expressing CDK11b^{p58} and/or a cell expressing splicing factor 3B subunit 1 (SF3B1) and/or 14-3-3γ binding fragment of CITED2 with at least one compound that potentially modulates the interaction of CITED2 with at least one molecule selected from the group consisting of CDK11b^{p58}, splicing factor 3B subunit 1 (SF3B1), and 14-3-3γ,
b) identifying a modulation of the interaction of CITED2 or the fragment thereof with at least one molecule selected from the group consisting of CDK11b^{p58}, splicing factor 3B subunit 1 (SF3B1), and 14-3-3γ in the presence of said at least one compound, compared to the absence of said at least one compound,
and c) identifying at least one compound of step b) as modulating, in particular specifically modulating, the mitotic cell cycle in cancer cells based on the modulation as identified in step b), wherein preferably the modulation is selected from a decrease or an increase of said interaction, preferably an inhibition of the interaction.

4. The method according to any one of claims 1 to 3, wherein the cancer cells are selected from colorectal cancer cells, gastric cancer cells, rectal cancer cells, breast cancer cells, cervical cancer cells, endocervical cancer cells, colon cancer cells, esophageal cancer cells, brain cancer cells, head and neck cancer cells, renal cancer cells, meningeal cancer cells, glioma, glioblastoma, lung cancer cells, mesothelioma, ovarian cancer cells, pancreatic cancer cells, neuroendocrine cancer cells, prostatic cancer cells, skin cancer cells, stomach cancer cells, thyroid cancer cells, uterine cancer cells, and testicular cancer cells, and are preferably selected from colorectal cancer cells.

5. The method according to any one of claims 1 to 4, wherein the identifying in step c) comprises determining of centrosome segregation and subsequent bipolar spindle assembly in the mammalian cell, preferably determining bipolar centrosome separation and/or the formation of chaotic monopolar mitotic spindles.

6. The method according to any one of claims 1 to 5, wherein the identifying in step c) further comprises determining tumor volume and/or tumor number per subject.

7. The method according to any one of claims 1 to 6, wherein said compound is selected from the group consisting of a peptide library, a combinatory library, a cell extract, a "small molecular drug", an antisense oligonucleotide, such as a gapmer antisense oligonucleotide, an siRNA, and an antibody, in particular an antibody against CITED2 or a fragment thereof, e.g. a F(ab)2-fragment, specifically interfering with the binding of CITED2 or the binding fragment thereof to at least one molecule selected from the group consisting of CDK11b^{p58}, splicing factor 3B subunit 1 (SF3B1), and 14-3-3γ and an antibody oligo conjugate (AOC) targeting CITED2 or antigen binding fragment oligo conjugate thereof.

8. The method according to any one of claims 1 to 7, further comprising a computational analysis and optimization of said compound based on the structure, in particular the three-dimensional and/or crystal structure of CITED2 and/or CDK11b^{p58} and/or splicing factor 3B subunit 1 (SF3B1) and/or 14-3-3γ.

9. A screening tool for screening a compound that modulates the expression, the stability, the biological activity and/or the interaction of CITED2 with at least one molecule selected from the group consisting of CDK11b^{p58}, splicing factor 3B subunit 1 (SF3B1), and 14-3-3γ, preferably according to a method according to any one of claims 1 to 8, comprising an isolated cell expressing, in particular recombinantly expressing, CITED2, and/or expressing an CDK11b^{p58} and/or splicing factor 3B subunit 1 (SF3B1) and/or 14-3-3γ binding fragment thereof, wherein said cell optionally expresses CDK11b^{p58} and/or an CITED2 binding fragment thereof, wherein preferably said CITED2 and/or CDK11b^{p58} and/or splicing factor 3B subunit 1 (SF3B1) and/or 14-3-3γ and/or the fragments thereof are immobilized and/or labeled.

10. A method for determining the level of proper centrosome segregation and subsequent bipolar spindle assembly in a sample obtained from a subject, comprising detecting the concentration of CITED2 in the sample, wherein a decrease of the concentration of CITED2 in the sample when compared to a control indicates an improper centrosome segregation and subsequent an increase of the formation of chaotic monopolar mitotic spindles in said sample.

11. A method for determining the level of CITED2 and/or CDK11b^{p58} and/or splicing factor 3B subunit 1 (SF3B1) and/or 14-3-3γ in a sample obtained from a subject, comprising detecting binding of CITED2 in the sample to at least one molecule selected from the group consisting of CDK11b^{p58}, splicing factor 3B subunit 1 (SF3B1) and 14-3-3γ, wherein a decrease of the binding, when compared to a control, indicates an improper centrosome segregation and a subsequent increase of the formation of chaotic monopolar mitotic spindles in said sample.

12. A method for detecting the progression of cancer cells and/or the risk for the progression of cancer cells in a sample, comprising performing a method according to claim 10 or 11, wherein a decrease of CITED2 and/or decrease of binding of CITED2 to at least one molecule selected from the group consisting of CDK11b^{p58}, splicing factor 3B subunit 1 (SF3B1) and 14-3-3γ in the sample is indicative for the formation of chaotic monopolar mitotic spindles in said sample and a decrease of the progression of cancer cells and/or the risk for the progression of cancer cells.

13. A method for manufacturing a pharmaceutical composition for treating or preventing cancer cells, comprising the step of performing the method according to any of claims 1 to 8, and formulating said compound as identified into a pharmaceutical composition, wherein said compound preferably is an CITED2 antisense oligonucleotide, such as a gapmer antisense oligonucleotide, an siRNA, or an antibody, in particular an antibody against CITED2 or fragment thereof, e.g. a F(ab)2-fragment, specifically interfering with the binding of CITED2 or the binding fragment thereof to at least one molecule selected from the group consisting of CDK11b^{p58}, splicing factor 3B subunit 1 (SF3B1), 14-3-3γ, and an antibody oligo conjugate (AOC) targeting CITED2 or antigen binding fragment oligo conjugate thereof

14. A pharmaceutical composition as produced according to claim 13, or a compound as identified with a method according to any of claims 1 to 8, preferably an CITED2 antisense oligonucleotide, such as a gapmer antisense oligonucleotide, an siRNA, or an antibody, in particular an antibody against CITED2 or fragment thereof, e.g. a F(ab)2-fragment, specifically interfering with the binding of CITED2 or the binding fragment thereof to at least one molecule selected from the group consisting of CDK11b^{p58}, splicing factor 3B subunit 1 (SF3B1), 14-3-3γ, and an antibody oligo conjugate (AOC) targeting CITED2 or antigen binding fragment oligo conjugate thereof for use in the prevention or treatment of a disease or condition caused by said cancer cells, such as, for example, selected from gastric cancer cells, colon cancer cells, rectal cancer cells, lung cancer and/or pancreatic cancer cells, wherein preferably the prevention or treatment is in combination with at least one checkpoint-inhibitor (ICI).

15. A method for monitoring the treatment of a disease or condition caused by cancer cells, such as, for example, gastric cancer cells, colon cancer cells, and/or rectal cancer cells in a subject, comprising performing the method according to claim 10 or 11 on a sample obtained from a subject before and after or during a treatment according to claim 14, and monitoring the treatment of the disease or condition based on the differences as detected between the samples.
